# EUROPEAN PATENT APPLICATION

(11) **EP 4 810 499 A1**
(43) Date of publication of application: **23.09.2026**
(21) Application number: 24890781.8
(22) Date of filing: 14.11.2024
(51) Int. Cl.: C07D 495/04, A61K 31/407, A61K 9/48, A61K 9/20, A61P 37/02

(54) **TREATMENT OF BEHÇET'S DISEASE**

(30) Priority: 14.11.2023 CN 202311515655
(71) Applicant: Ganzhou Hemay Pharmaceutical, Co., Ltd, Jiangxi Province (CN)
(72) Inventor: ZHANG, Hesheng, Ganzhou, Jiangxi 341600 (CN); HUO, Aihong, Ganzhou, Jiangxi 341600 (CN); CHEN, Yingwei, Ganzhou, Jiangxi 341600 (CN); SONG, Dan, Ganzhou, Jiangxi 341600 (CN); ZHANG, Yumei, Ganzhou, Jiangxi 341600 (CN)
(74) Representative: Mewburn Ellis LLP
(86) International application number: PCT/CN2024/132105
(87) International publication number: WO 2025/103426

(57) **Abstract**

Disclosed is a method for treating Behçet's disease. The method comprises administering to a subject in need a therapeutically effective amount of a compound represented by formula (I), a stereoisomer thereof, or a pharmaceutically acceptable salt thereof.

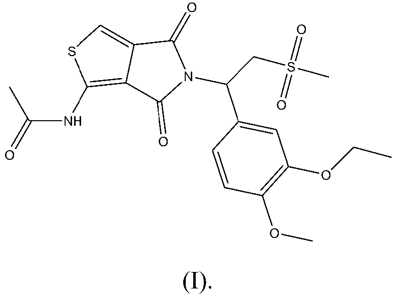

## Description

### CROSS-REFERENCE TO RELATED APPLICATION

The present disclosure claims the full benefits of Chinese Patent Application No. 202311515655.8, filed on November 14, 2023, with the State Intellectual Property Office of the People's Republic of China, entitled "Treatment of Behçet's Disease", which is incorporated herein by reference in its entirety.

### TECHNICAL FIELD

The present disclosure generally relates to the field of medicine, and more specifically, to the treatment of Behçet's syndrome.

### BACKGROUND

Behçet's disease (BD), also known as Behçet's syndrome, is a chronic systemic vascular inflammatory disease. Its major manifestations include recurrent oral ulcers, genital ulcers, ophthalmia and skin lesions, and said disease can also involve organs such as the blood vessels, heart, nervous system, digestive tract, joints, lungs, kidneys and epididymis. Behçet's disease exhibits regional differences, with a relatively high incidence in East Asia, the Middle East and the Mediterranean region, and is also referred to as the Silk Road disease. It predominantly affects individuals aged 16 to 40 years. Overall, the male-to-female ratio is approximately equal.

Currently, there are no specific biomarkers or histopathological features for Behçet's disease. Diagnosis is mainly based on clinical symptoms. There is no specific treatment for Behçet's disease, and the primary purposes of treatment are to relieve symptoms, reduce recurrence, delay the progression of the disease and prevent serious complications. Mucocutaneous manifestations typically follow a recurrent, remitting and self-limiting process. Although these manifestations are not life-threatening, they can exert a negative impact on the quality of life. Patients suffering from Behçet's disease often have impaired or disordered immune function, thereby increasing a risk of infection with pathogens. Long-term administration of currently available therapeutic drugs for Behçet's disease may further reduce immune competence in patients suffering from Behçet's disease, thereby further increasing the risk of infection with pathogens. Among such pathogens, *Mycobacterium tuberculosis* is a common infectious bacterium encountered during treatment. Approximately one-third of the global population is infected with *Mycobacterium tuberculosis*, and 90% of infected individuals can carry the pathogen persistently for a long time and develop latent tuberculosis infection. Latent tuberculosis infection serves as a breeding ground for the development of active tuberculosis, and patients with latent tuberculosis infection exhibit highly uncertain disease progression outcome, representing a concealed critical risk in tuberculosis control. Impaired immune function in patients with latent tuberculosis infection tends to facilitate the progression from latent tuberculosis to active tuberculosis. It is of profound significance and impact to focus on the special patient population with latent tuberculosis infection during treatment, provide appropriate therapeutic regimens for patients with Behçet's disease as well as patients with Behçet's disease accompanied by latent tuberculosis infection or a history of tuberculosis, improve efficacy, reduce adverse reactions and the potential risk of progression to active tuberculosis, and strive for greater benefits for such patients.

### SUMMARY OF THE INVENTION

In one aspect, the present disclosure relates to a method for treating Behçet's syndrome, comprising administering to a subject in need thereof a therapeutically effective amount of a compound of formula (I), a stereoisomer thereof, or a pharmaceutically acceptable salt thereof,

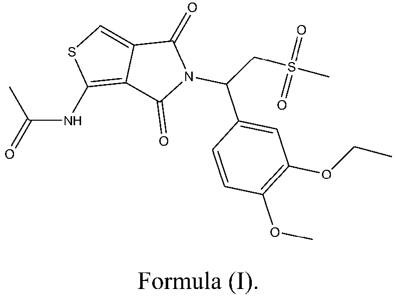

In one aspect, the present disclosure relates to a method for treating oral ulcers associated with Behçet's syndrome, comprising administering to a subject in need thereof a therapeutically effective amount of a compound of formula (I), a stereoisomer thereof, or a pharmaceutically acceptable salt thereof,

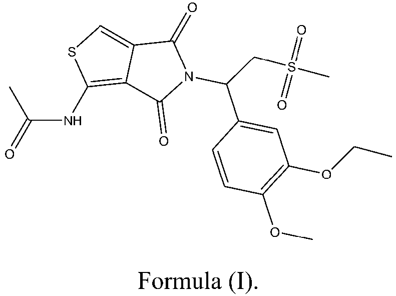

In one aspect, the present disclosure relates to a method for treating genital ulcers associated with Behçet's syndrome, comprising administering to a subject in need thereof a therapeutically effective amount of a compound of formula (I), a stereoisomer thereof, or a pharmaceutically acceptable salt thereof,

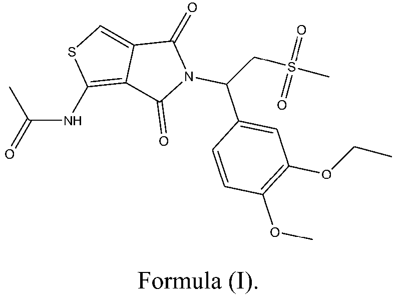

In another aspect, the present disclosure relates to a compound of formula (I), a stereoisomer thereof, or a pharmaceutically acceptable salt thereof for use in the treatment of Behçet's syndrome in a subject,

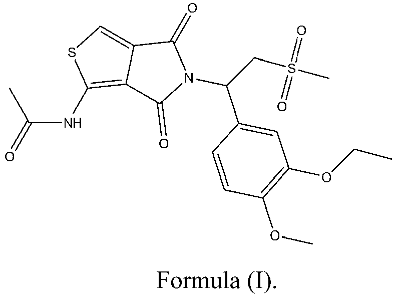

In another aspect, the present disclosure relates to a compound of formula (I), a stereoisomer thereof, or a pharmaceutically acceptable salt thereof for use in the treatment of oral ulcers associated with Behçet's syndrome in a subject,

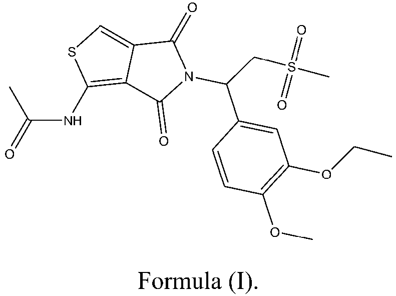

In another aspect, the present disclosure relates to a compound of formula (I), a stereoisomer thereof, or a pharmaceutically acceptable salt thereof for use in the treatment of genital ulcers associated with Behçet's syndrome in a subject,

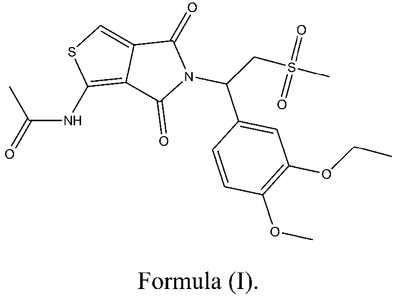

In still another aspect, the present disclosure relates to a use of a compound of formula (I), a stereoisomer thereof, or a pharmaceutically acceptable salt thereof in the preparation of a medicament for the treatment of Behçet's syndrome in a subject,

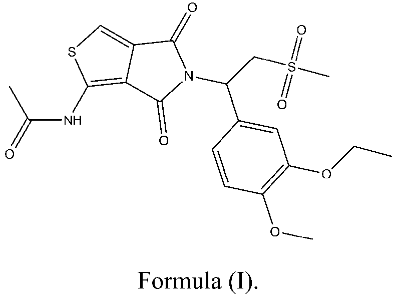

In still another aspect, the present disclosure relates to a use of a compound of formula (I), a stereoisomer thereof, or a pharmaceutically acceptable salt thereof in the preparation of a medicament for the treatment of oral ulcers associated with Behçet's syndrome in a subject,

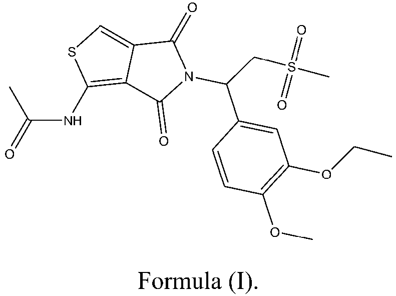

In still another aspect, the present disclosure relates to a use of a compound of formula (I), a stereoisomer thereof, or a pharmaceutically acceptable salt thereof in the preparation of a medicament for the treatment of genital ulcers associated with Behçet's syndrome in a subject,

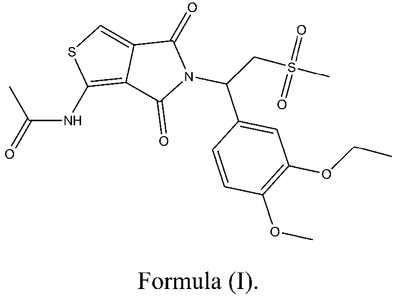

In yet another aspect, the present disclosure relates to a pharmaceutical composition for use in the treatment of Behçet's syndrome in a subject, comprising a compound of formula (I), a stereoisomer thereof, or a pharmaceutically acceptable salt thereof, and a pharmaceutically acceptable carrier, diluent or excipient,

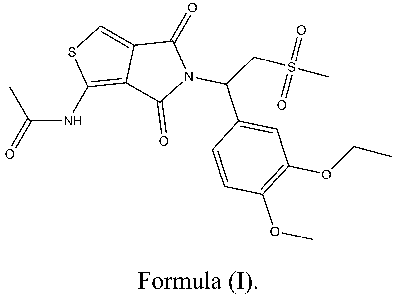

In yet another aspect, the present disclosure relates to a pharmaceutical composition for use in the treatment of oral ulcers associated with Behçet's syndrome in a subject, comprising a compound of formula (I), a stereoisomer thereof, or a pharmaceutically acceptable salt thereof, and a pharmaceutically acceptable carrier, diluent or excipient,

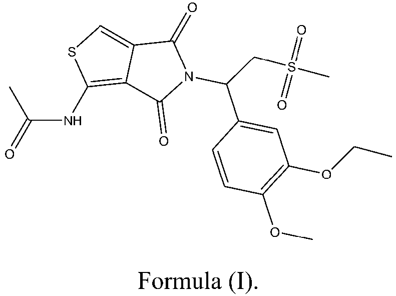

In yet another aspect, the present disclosure relates to a pharmaceutical composition for use in the treatment of genital ulcers associated with Behçet's syndrome in a subject, comprising a compound of formula (I), a stereoisomer thereof, or a pharmaceutically acceptable salt thereof, and a pharmaceutically acceptable carrier, diluent or excipient,

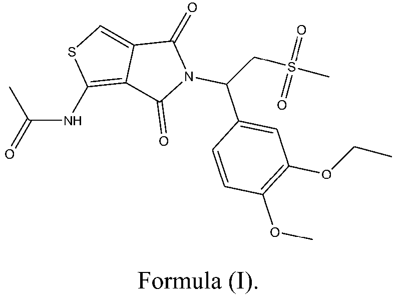

### DETAILED DESCRIPTION OF THE INVENTION

In the following description, certain specific details are set forth to provide a thorough understanding of various embodiments of the disclosure. However, a person skilled in the related art would recognize that the embodiments can still be practiced by using other methods, components, materials, etc., without employing one or more of these specific details.

Unless required otherwise in the present disclosure, throughout the specification and the appended claims, the words "comprising," "including," "containing," and "having" are to be construed in an open, inclusive sense, that is, as "including but not limited to".

When used in the present disclosure and the appended claims, a singular designation without a quantity indication includes a plural designation unless stated otherwise in the context.

Reference throughout this specification to "one embodiment", "an embodiment", "in another embodiment", and "in some embodiments" means that a specific reference element, structure, or characteristic described in connection with the embodiment is included in at least one embodiment of the present disclosure. Thus, the appearances of the phrases "in one embodiment", "in an embodiment", "in another embodiment", and "in some embodiments" in various places throughout this specification are not necessarily all referring to the same embodiment. Furthermore, specific elements, structures, or features may be combined in any suitable manner in one or more embodiments.

It should be understood that the singular articles "a", "an" and "the" used in the specification and the appended claims of the present disclosure include plural references unless the context clearly dictates otherwise. Therefore, for example, reference to a pharmaceutical composition comprising "an excipient" includes a pharmaceutical composition comprising one excipient, or a pharmaceutical composition comprising two or more excipients.

### Definition

Therefore, unless stated otherwise, the following terms used in the present specification and appended claims shall have the following meanings.

In the present disclosure, the term "Behçet's syndrome" is also referred to as Behçet's disease, oral-ocular-genital triad, and the like. It is a chronic systemic vascular inflammatory disease, mainly manifested as recurrent oral ulcers, genital ulcers, ophthalmia and skin lesions. It may also involve organs such as the blood vessels, nervous system, digestive tract, joints, lungs, kidneys and epididymis. Most patients have a favorable prognosis, while those with involvement of the eyes, central nervous system and large blood vessels have a poor prognosis. Currently, the International Diagnostic (Classification) Criteria for Behçet's disease established in 2013 (ICBD-2013) by the International Study Group for Behçet's Disease (ISG) is adopted clinically: oral ulcers, genital ulcers and ocular lesions (anterior uveitis, posterior uveitis, or retinal vasculitis) are each scored 2 points; skin lesions (pseudofolliculitis, skin ulcers, or erythema nodosum), central nervous system involvement, and vascular lesions (arterial thrombosis, large venous thrombosis, phlebitis, or superficial phlebitis) are each scored 1 point; and a positive pathergy test is scored 1 point. A case with a total score of 4 or higher indicates a diagnosis of Behçet's syndrome.

In the present disclosure, the term "complete remission of oral ulcers" refers to the time when complete healing of the oral ulcer first occurs, and ulcer healing is defined as complete flattening of the ulcer surface, absence of hyperemia, and absence of pain.

In the present disclosure, the term "visual analogue scale score for oral ulcer pain" is used for pain assessment. It is widely used in clinical practice in China. The basic method is to use a sliding scale approximately 10 cm in length, with 10 scales marked on one side and two ends labeled as the "0" score end and the "10" score end, respectively, where a score of 0 indicates no pain and a score of 10 represents the most severe unbearable pain.

In the present disclosure, the term "Physician's Global Assessment" refers to the overall assessment of BD-related skin lesions in subjects performed by investigators.

In the present disclosure, the term "BD Current Activity Assessment Scale" is developed by the International Behçet's Disease Scientific Committee, and is used for daily monitoring of subjects and clinical studies. The questionnaire is used by investigators to assess subjects and quantify the clinical manifestations of the disease over the past 4 weeks on a 12-point scale, with a higher score indicating a higher level of disease activity. BDCAF is a validated instrument for assessing mucocutaneous and articular lesions of BD, as well as for assessing general symptoms and non-mucocutaneous clinical manifestations of BD.

In the present disclosure, the term "Ritchie Articular Index Score" is graded into four levels (mild, slight, moderate, and severe), with a higher score indicating a poorer joint status in subjects.

In the present disclosure, the term "BVAS" refers to the Birmingham Vasculitis Activity Score. Assessments of subjects shall be performed by investigators at the specified study visits. BVAS assesses new manifestations or exacerbations caused by vasculitis within 4 weeks prior to the assessment date. This score rates multiple systems of subjects, including general condition (maximum 3 points), skin (maximum 6 points), mucosa/eyes (maximum 6 points), ear, nose and throat (maximum 6 points), chest (maximum 6 points), cardiovascular system (maximum 6 points), abdomen (maximum 9 points), kidney (maximum 12 points), and the nervous system (maximum 9 points). Each system has a maximum score limit, with a total maximum score of 63 points. A score of 15 or higher indicates disease activity.

In the present disclosure, the term "AUC" is calculated using the linear trapezoidal method. The AUC of a subject is defined as the sum of the trapezoidal areas between every two adjacent assessment visits for oral ulcers.

An Analysis of Covariance (ANCOVA) model is applied, with treatment group and subject gender as fixed factor variables, and the number of oral ulcers at baseline as a covariate, to calculate the between-group difference in least squares means of AUC and the corresponding 95% confidence interval (95% C.I.). For the calculation of AUC, multiple imputation is performed to process missing data on the number of oral ulcers in subjects.

In the present disclosure, the term "HADS" refers to the Hospital Anxiety and Depression Scale, which is used for screening anxiety and depressive symptoms in patients in general hospitals. Featuring simplicity, conciseness and practicality, the questionnaire has been extensively studied in routine medical settings. The questionnaire contains 7 anxiety-related items (A) and 7 depression-related items (D). Responses to all items are rated on a 4-point scale (0-3 points). The total score for anxiety or depression is the sum of the scores for individual responses to the anxiety or depression-related items, ranging from 0 to 21 points. Scores of 0 to 7 indicate the absence of anxiety or depression; scores of 8 to 10 indicate mild anxiety or depression; scores of 11 to 14 indicate moderate anxiety or depression; and scores of 15 to 21 indicate severe anxiety or depression.

In the present disclosure, there are two commonly used classification methods for adverse events occurring in clinical trials. One classification method categorizes adverse reactions as mild, moderate, and severe. "Mild" means that said adverse reaction is usually transient, generally does not affect the subjects' activities of daily living, and may require only minimal treatment. "Moderate" means that said adverse reaction causes discomfort to the subjects, affects activities of daily living, and usually requires treatment for relief, but does not pose a risk of substantial or permanent harm to the subject. "Severe" means that said adverse reaction significantly affects the subjects' activities of daily living or seriously deteriorates their clinical condition, requiring intensive treatment and intervention. The other classification method categorizes adverse events as Grade 1 to Grade 5 with reference to the Common Terminology Criteria for Adverse Events (CTCAE). Grade 1: mild, no or mild clinical symptoms; only clinical or diagnostic observations; no treatment required. Grade 2: moderate, requires minor, local or non-invasive treatment, limitation in age-appropriate instrumental Activities of Daily Living (ADL). Instrumental Activities of Daily Living refer to cooking, clothing purchasing, telephone use, financial management, etc. Grade 3: severe or medically significant, but not immediately life-threatening; results in hospitalization or prolonged hospitalization; disability; limitations in basic activities of daily living. Basic Activities of Daily Living refer to bathing, dressing, eating, personal hygiene, medication administration, etc., without bedridden status. Grade 4: life-threatening, requires urgent intervention. Grade 5: death related to the adverse event.

In the present disclosure, the term "latent tuberculosis infection (LTBI)" means that an organism is infected with *Mycobacterium tuberculosis*, without development of clinical tuberculosis, absence of clinical symptoms, and no bacteriological or imaging evidence of active tuberculosis. Diagnostic criteria for latent tuberculosis include a positive T-Cell Spot Assay (T-SPOT) for *Mycobacterium tuberculosis* infection and no clinical manifestations of active tuberculosis.

In the present disclosure, the term "activation" refers to a patient's *Mycobacterium tuberculosis* infection status transition from latent tuberculosis infection to active tuberculosis infection; that is, the patient's *Mycobacterium tuberculosis* infection status progresses to an active stage, and the patient is in an active tuberculosis state, and requires standardized and intensive treatment.

In the present disclosure, the term "titrated dosing" refers to the gradual attainment of a desired drug level by administration of a low dose of the drug.

In the present disclosure, the term "compound of formula (I)" refers to N-[5-[1-(3-ethoxy-4-methoxyphenyl)-2-(methylsulfonyl)ethyl]-4,6-dioxo-5,6-dihydro-4H-thieno[3,4-c]pyrrol-1-yl]acetamide, the structural formula of which is shown below:

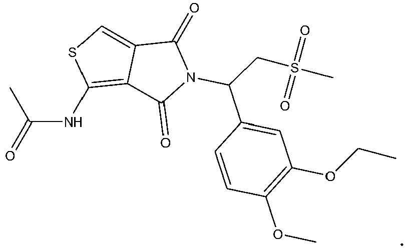

In the present disclosure, the term "a stereoisomer of formula (I)" refers to (S)-N-[5-[1-(3-ethoxy-4-methoxyphenyl)-2-(methylsulfonyl)ethyl]-4,6-dioxo-5,6-dihydro-4H-thieno[3,4-c]pyrrol-1-yl]acetamide, the structural formula of which is shown below:

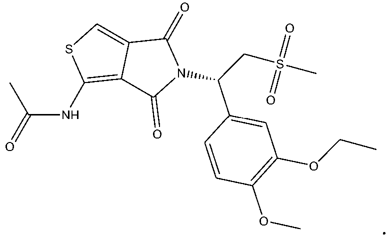

In the present disclosure, the term "pharmaceutically acceptable" means that a carrier, vehicle, diluent, excipient, and/or salt is compatible with other components of the formulation and not deleterious to the recipient thereof.

In the present disclosure, the term "pharmaceutically acceptable carrier, diluent, or excipient" includes, but is not limited to, any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier in various forms approved by the United States Food and Drug Administration for use in humans or animals, which has no adverse effects on the pharmaceutical composition.

In the present disclosure, the term "carrier" is defined as a compound that facilitates the introduction of a compound into a cell or tissue. For example, dimethyl sulfoxide (DMSO) is commonly used as a carrier due to its ability to readily introduce certain organic compounds into a cell or tissue of an organism.

In the present disclosure, the term "pharmaceutically acceptable salt" includes "an acceptable acid addition salt" and "an acceptable base addition salt".

In the present disclosure, the term "acceptable acid addition salt" refers to those salts which retain the biological effectiveness and properties of the free bases, which are biologically or otherwise desirable, and are formed with inorganic or organic acids. The inorganic acids include, but are not limited to, hydrochloric acid, hydrobromic acid, sulfuric acid, nitric acid, phosphoric acid, and the like. The organic acid include, but are not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

In the present disclosure, the term "acceptable base addition salt" refers to those salts which retain the biological effectiveness and properties of the free acids, which are biologically or otherwise desirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. In certain embodiments, inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like. In certain embodiments, organic bases are isopropylamine, diethylamine, ethanolamine, trimethylamine, dicyclohexylamine, choline and caffeine.

In the present disclosure, the term "mammal" refers to an animal including, for example, a dog, cat, cow, sheep, horse, and human. In some embodiments, the mammal is a human.

In the present disclosure, the term "pharmaceutical composition" refers to a formulation of a compound of formula (I) or a pharmaceutically acceptable salt thereof described in the present disclosure and a medium generally accepted in the art for the delivery of the biologically active compound to mammals, e.g., humans. Such a medium includes all pharmaceutically acceptable carriers, diluents or excipients therefor.

In the present disclosure, the term "therapeutically effective amount" refers to an amount of a compound or a combination of compounds that ameliorates, attenuates, or eliminates a specific disease or condition and the symptoms thereof, or prevents or delays such specific disease or condition or the onset of the symptoms thereof. The amount of the compound of formula (I) described in the present disclosure which constitutes a "therapeutically effective amount" will vary depending on the compound, the condition and its severity, and the age and weight of the mammal to be treated, but the amount of the compound described in the present disclosure can be determined routinely by one of ordinary skill in the art having regard to his own knowledge and to the present disclosure.

As used in the present disclosure, "treating" or "treatment" covers the treatment of the disease or condition of interest in a mammal, such as a human, having the disease or disorder of interest, and includes:
(i) preventing the occurrence of a disease or condition in a mammal, especially where such mammal is susceptible to said disease or condition but has not yet been diagnosed as suffering therefrom;
(ii) inhibiting the disease or condition, i.e., arresting its development; or
(iii) relieving the disease or condition, i.e., causing regression or resulting in no progression of the disease or condition.

In the present disclosure, the term "unit dose" refers to a dosage for a single application. For example, for a tablet, a unit dose refers to the dosage of one tablet.

The compound or a pharmaceutically acceptable salt thereof of the present disclosure may contain one or more asymmetric centers, and thus may give rise to enantiomers, diastereomers, and other stereoisomeric forms, which may be defined as (R)- or (S)- in terms of absolute stereochemistry, or as (D)- or (L)- for amino acids. The present disclosure is intended to include all such possible isomers, as well as racemic and optically pure forms thereof. Optically active (+) and (-), (R)- and (S)-, or (D)- and (L)- isomers may be prepared using chiral synthons or chiral reagents, or may be resolved using conventional techniques, such as HPLC with a chiral column. When the compound described herein contains olefinic double bonds or other centers of geometric asymmetry, unless otherwise specified, it is intended that the compound includes both E and Z geometric isomers. Likewise, all tautomeric forms are also intended to be included.

In the present disclosure, the term "stereoisomer" refers to compounds consisting of identical atoms bonded by identical bonds, but having distinct three-dimensional structures that are not interchangeable. The present disclosure encompasses all stereoisomers and mixtures thereof.

### Detailed Description

In one aspect, the present disclosure relates to a method for treating Behçet's syndrome, comprising administering to a subject in need thereof a therapeutically effective amount of a compound of formula (I), a stereoisomer thereof, or a pharmaceutically acceptable salt thereof,

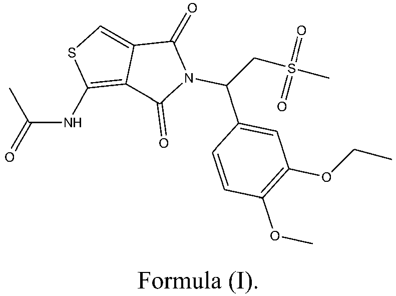

In one aspect, the present disclosure relates to a method for treating oral ulcers associated with Behçet's syndrome, comprising administering to a subject in need thereof a therapeutically effective amount of a compound of formula (I), a stereoisomer thereof, or a pharmaceutically acceptable salt thereof,

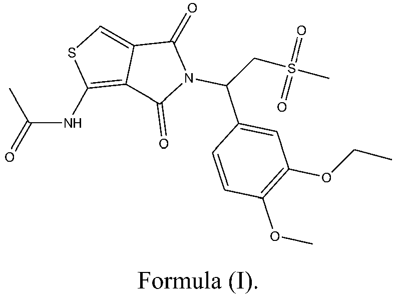

In one aspect, the present disclosure relates to a method for treating genital ulcers associated with Behçet's syndrome, comprising administering to a subject in need thereof a therapeutically effective amount of a compound of formula (I), a stereoisomer thereof, or a pharmaceutically acceptable salt thereof,

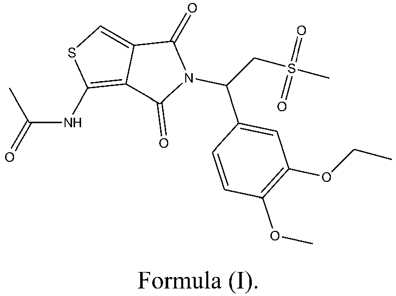

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg or 180 mg.

In some embodiments, the compound of formula (I), a stereoisomer thereof, or a pharmaceutically acceptable salt thereof is administered to the subject daily at a dose of 60 mg to 150 mg.

In some embodiments, the compound of formula (I), a stereoisomer thereof, or a pharmaceutically acceptable salt thereof is administered to the subject daily at a dose of 90 mg to 150 mg.

In some embodiments, the compound of formula (I), a stereoisomer thereof, or a pharmaceutically acceptable salt thereof is administered to the subject daily at a dose of 30 mg, 45 mg, 60 mg, 90 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof is administered to the subject twice daily at a dose of 45 mg to 60 mg each time.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg and 150 mg.

In some embodiments, the compound of formula (I), a stereoisomer thereof, or a pharmaceutically acceptable salt thereof has a unit dosage of 15 mg to 75 mg.

In some embodiments, the compound of formula (I), a stereoisomer thereof, or a pharmaceutically acceptable salt thereof has a unit dosage of 15 mg to 150 mg.

In some embodiments, the compound of formula (I), a stereoisomer thereof, or a pharmaceutically acceptable salt thereof has a unit dosage of 15 mg, 30 mg, 60 mg and 75 mg.

In some embodiments, the compound of formula (I), a stereoisomer thereof, or a pharmaceutically acceptable salt thereof has a unit dosage of 15 mg, 30 mg, 45 mg, 60 mg, 75 mg, 90 mg, 120 mg and 150 mg.

In some embodiments, the compound of formula (I), a stereoisomer thereof, or a pharmaceutically acceptable salt thereof is administered to the subject in a tablet or capsule form.

In some embodiments, the compound of formula (I), a stereoisomer thereof, or a pharmaceutically acceptable salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse, and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the compound of formula (I), a stereoisomer thereof, or a pharmaceutically acceptable salt thereof of the present disclosure has good tolerability when used in the treatment of Behçet's syndrome.

In some embodiments, the compound of formula (I), a stereoisomer thereof, or a pharmaceutically acceptable salt thereof of the present disclosure has good compliance when used in the treatment of Behçet's syndrome.

In some embodiments, the compound of formula (I), a stereoisomer thereof, or a pharmaceutically acceptable salt thereof of the present disclosure does not require titrated dosing when used in the treatment of Behçet's syndrome.

In some embodiments, the compound of formula (I), a stereoisomer thereof, or a pharmaceutically acceptable salt thereof of the present disclosure has a rapid onset of action when used in the treatment of Behçet's syndrome.

In some embodiments, the compound of formula (I), a stereoisomer thereof, or a pharmaceutically acceptable salt thereof of the present disclosure has excellent therapeutic effects when used in the treatment of Behçet's syndrome.

In some embodiments, the compound of formula (I), a stereoisomer thereof, or a pharmaceutically acceptable salt thereof of the present disclosure has favorable safety when used in the treatment of Behçet's syndrome.

In some embodiments, the compound of formula (I), a stereoisomer thereof, or a pharmaceutically acceptable salt thereof of the present disclosure can be used in the treatment of Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome.

In some embodiments, the compound of formula (I), a stereoisomer thereof, or a pharmaceutically acceptable salt thereof of the present disclosure can reduce the area under the curve of oral ulcers when used in the treatment of Behçet's syndrome.

In some embodiments, the compound of formula (I), a stereoisomer thereof, or a pharmaceutically acceptable salt thereof of the present disclosure can reduce the number of oral ulcers when used in the treatment of Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome.

In some embodiments, the compound of formula (I), a stereoisomer thereof, or a pharmaceutically acceptable salt thereof of the present disclosure can alleviate oral ulcer pain when used in the treatment of Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome.

In some embodiments, the compound of formula (I), a stereoisomer thereof, or a pharmaceutically acceptable salt thereof of the present disclosure can improve mucocutaneous involvement in the subject when used in the treatment of Behçet's syndrome.

In some embodiments, the compound of formula (I), a stereoisomer thereof, or a pharmaceutically acceptable salt thereof of the present disclosure can improve joint involvement in the subject when used in the treatment of Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome.

In some embodiments, the compound of formula (I), a stereoisomer thereof, or a pharmaceutically acceptable salt thereof of the present disclosure does not diminish immunity in the subject when used in the treatment of Behçet's syndrome.

In some embodiments, the compound of formula (I), a stereoisomer thereof, or a pharmaceutically acceptable salt thereof of the present disclosure has good tolerability when used in the treatment of oral ulcers associated with Behçet's syndrome.

In some embodiments, the compound of formula (I), a stereoisomer thereof, or a pharmaceutically acceptable salt thereof of the present disclosure has good compliance when used in the treatment of oral ulcers associated with Behçet's syndrome.

In some embodiments, the compound of formula (I), a stereoisomer thereof, or a pharmaceutically acceptable salt thereof of the present disclosure does not require titrated dosing when used in the treatment of oral ulcers associated with Behçet's syndrome.

In some embodiments, the compound of formula (I), a stereoisomer thereof, or a pharmaceutically acceptable salt thereof of the present disclosure has a rapid onset of action when used in the treatment of oral ulcers associated with Behçet's syndrome.

In some embodiments, the compound of formula (I), a stereoisomer thereof, or a pharmaceutically acceptable salt thereof of the present disclosure has excellent therapeutic effects when used in the treatment of oral ulcers associated with Behçet's syndrome.

In some embodiments, the compound of formula (I), a stereoisomer thereof, or a pharmaceutically acceptable salt thereof of the present disclosure has favorable safety when used in the treatment of oral ulcers associated with Behçet's syndrome.

In some embodiments, the compound of formula (I), a stereoisomer thereof, or a pharmaceutically acceptable salt thereof of the present disclosure can be used in the treatment of oral ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of oral ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of oral ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of oral ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of oral ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of oral ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of oral ulcers associated with Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of oral ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of oral ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of oral ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of oral ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of oral ulcers associated with Behçet's syndrome.

In some embodiments, the compound of formula (I), a stereoisomer thereof, or a pharmaceutically acceptable salt thereof of the present disclosure has good tolerability when used in the treatment of genital ulcers associated with Behçet's syndrome.

In some embodiments, the compound of formula (I), a stereoisomer thereof, or a pharmaceutically acceptable salt thereof of the present disclosure has good compliance when used in the treatment of genital ulcers associated with Behçet's syndrome.

In some embodiments, the compound of formula (I), a stereoisomer thereof, or a pharmaceutically acceptable salt thereof of the present disclosure does not require titrated dosing when used in the treatment of genital ulcers associated with Behçet's syndrome.

In some embodiments, the compound of formula (I), a stereoisomer thereof, or a pharmaceutically acceptable salt thereof of the present disclosure has a rapid onset of action when used in the treatment of genital ulcers associated with Behçet's syndrome.

In some embodiments, the compound of formula (I), a stereoisomer thereof, or a pharmaceutically acceptable salt thereof of the present disclosure has excellent therapeutic effects when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of genital ulcers associated with Behçet's syndrome.

In some embodiments, the compound of formula (I), a stereoisomer thereof, or a pharmaceutically acceptable salt thereof of the present disclosure can be used in the treatment of genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of genital ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of genital ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of genital ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of genital ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of genital ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of genital ulcers associated with Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of genital ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of genital ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of genital ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of genital ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good tolerability when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good compliance when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not require titrated dosing when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has a rapid onset of action when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can be used in the treatment of Behçet's syndrome, oral ulcers associated with Behçet's syndrome, or genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject having a medical history of pulmonary tuberculosis infection, tuberculosis, and/or tuberculous pleurisy, oral ulcers associated with Behçet's syndrome in a subject suffering from latent tuberculosis infection, or genital ulcers associated with Behçet's syndrome in a subject suffering from latent tuberculosis infection, and pulmonary tuberculosis infection, tuberculosis, and/or tuberculous pleurisy as well as latent tuberculosis infection is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can reduce the area under the curve of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can reduce the number of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can alleviate oral ulcer pain when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve mucocutaneous involvement in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve joint involvement in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not diminish immunity in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In another aspect, the present disclosure relates to a compound of formula (I), a stereoisomer thereof, or a pharmaceutically acceptable salt thereof for use in the treatment of Behçet's syndrome in a subject,

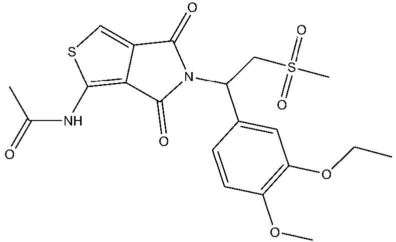

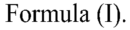

In another aspect, the present disclosure relates to a compound of formula (I), a stereoisomer thereof, or a pharmaceutically acceptable salt thereof for use in the treatment of oral ulcers associated with Behçet's syndrome in a subject,

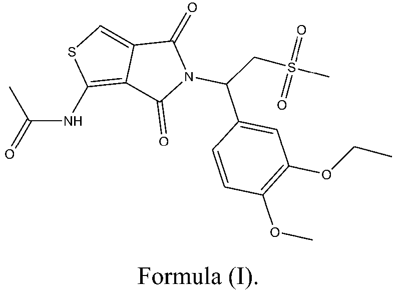

In another aspect, the present disclosure relates to a compound of formula (I), a stereoisomer thereof, or a pharmaceutically acceptable salt thereof for use in the treatment of genital ulcers associated with Behçet's syndrome in a subject,

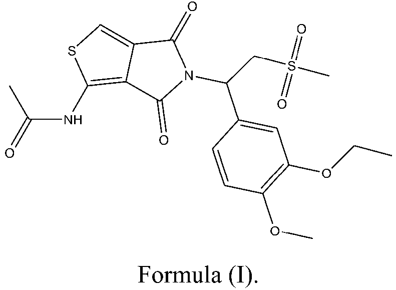

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg or 180 mg.

In some embodiments, the compound of formula (I), a stereoisomer thereof, or a pharmaceutically acceptable salt thereof is administered to the subject daily at a dose of 60 mg to 150 mg.

In some embodiments, the compound of formula (I), a stereoisomer thereof, or a pharmaceutically acceptable salt thereof is administered to the subject daily at a dose of 90 mg to 150 mg.

In some embodiments, the compound of formula (I), a stereoisomer thereof, or a pharmaceutically acceptable salt thereof is administered to the subject daily at a dose of 30 mg, 45 mg, 60 mg, 90 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof is administered to the subject twice daily at a dose of 45 mg to 60 mg each time.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg and 150 mg.

In some embodiments, the compound of formula (I), a stereoisomer thereof, or a pharmaceutically acceptable salt thereof has a unit dosage of 15 mg to 75 mg.

In some embodiments, the compound of formula (I), a stereoisomer thereof, or a pharmaceutically acceptable salt thereof has a unit dosage of 15 mg to 150 mg.

In some embodiments, an exemplary unit dosage of the compound of formula (I), a stereoisomer thereof, or a pharmaceutically acceptable salt thereof of the present disclosure includes, but is not limited to, 15 mg, 30 mg, 60 mg, and 75 mg.

In some embodiments, the compound of formula (I), a stereoisomer thereof, or a pharmaceutically acceptable salt thereof has a unit dosage of 15 mg, 30 mg, 45 mg, 60 mg, 75 mg, 90 mg, 120 mg and 150 mg.

In some embodiments, the compound of formula (I), a stereoisomer thereof, or a pharmaceutically acceptable salt thereof is administered to the subject in a tablet or capsule form.

In some embodiments, the compound of formula (I), a stereoisomer thereof, or a pharmaceutically acceptable salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse, and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the compound of formula (I), a stereoisomer thereof, or a pharmaceutically acceptable salt thereof of the present disclosure has good tolerability when used in the treatment of Behçet's syndrome.

In some embodiments, the compound of formula (I), a stereoisomer thereof, or a pharmaceutically acceptable salt thereof of the present disclosure has good compliance when used in the treatment of Behçet's syndrome.

In some embodiments, the compound of formula (I), a stereoisomer thereof, or a pharmaceutically acceptable salt thereof of the present disclosure does not require titrated dosing when used in the treatment of Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of Behçet's syndrome.

In some embodiments, the compound of formula (I), a stereoisomer thereof, or a pharmaceutically acceptable salt thereof of the present disclosure has excellent therapeutic effects when used in the treatment of Behçet's syndrome.

In some embodiments, the compound of formula (I), a stereoisomer thereof, or a pharmaceutically acceptable salt thereof of the present disclosure has favorable safety when used in the treatment of Behçet's syndrome.

In some embodiments, the compound of formula (I), a stereoisomer thereof, or a pharmaceutically acceptable salt thereof of the present disclosure can be used in the treatment of Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome.

In some embodiments, the compound of formula (I), a stereoisomer thereof, or a pharmaceutically acceptable salt thereof of the present disclosure can reduce the area under the curve of oral ulcers when used in the treatment of Behçet's syndrome.

In some embodiments, the compound of formula (I), a stereoisomer thereof, or a pharmaceutically acceptable salt thereof of the present disclosure can reduce the number of oral ulcers when used in the treatment of Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome.

In some embodiments, the compound of formula (I), a stereoisomer thereof, or a pharmaceutically acceptable salt thereof of the present disclosure can alleviate oral ulcer pain when used in the treatment of Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome.

In some embodiments, the compound of formula (I), a stereoisomer thereof, or a pharmaceutically acceptable salt thereof of the present disclosure can improve mucocutaneous involvement in the subject when used in the treatment of Behçet's syndrome.

In some embodiments, the compound of formula (I), a stereoisomer thereof, or a pharmaceutically acceptable salt thereof of the present disclosure can improve joint involvement in the subject when used in the treatment of Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome.

In some embodiments, the compound of formula (I), a stereoisomer thereof, or a pharmaceutically acceptable salt thereof of the present disclosure does not diminish immunity in the subject when used in the treatment of Behçet's syndrome.

In some embodiments, the compound of formula (I), a stereoisomer thereof, or a pharmaceutically acceptable salt thereof of the present disclosure has good tolerability when used in the treatment of oral ulcers associated with Behçet's syndrome.

In some embodiments, the compound of formula (I), a stereoisomer thereof, or a pharmaceutically acceptable salt thereof of the present disclosure has good compliance when used in the treatment of oral ulcers associated with Behçet's syndrome.

In some embodiments, the compound of formula (I), a stereoisomer thereof, or a pharmaceutically acceptable salt thereof of the present disclosure does not require titrated dosing when used in the treatment of oral ulcers associated with Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of oral ulcers associated with Behçet's syndrome.

In some embodiments, the compound of formula (I), a stereoisomer thereof, or a pharmaceutically acceptable salt thereof of the present disclosure has excellent therapeutic effects when used in the treatment of oral ulcers associated with Behçet's syndrome.

In some embodiments, the compound of formula (I), a stereoisomer thereof, or a pharmaceutically acceptable salt thereof of the present disclosure has favorable safety when used in the treatment of oral ulcers associated with Behçet's syndrome.

In some embodiments, the compound of formula (I), a stereoisomer thereof, or a pharmaceutically acceptable salt thereof of the present disclosure can be used in the treatment of oral ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of oral ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of oral ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of oral ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of oral ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of oral ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of oral ulcers associated with Behçet's syndrome.

In some embodiments, the compound of formula (I), a stereoisomer thereof, or a pharmaceutically acceptable salt thereof of the present disclosure can improve related genital ulcers in the subject, and/or alleviate genital ulcer pain when used in the treatment of oral ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of oral ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of oral ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of oral ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of oral ulcers associated with Behçet's syndrome.

In some embodiments, the compound of formula (I), a stereoisomer thereof, or a pharmaceutically acceptable salt thereof of the present disclosure has good tolerability when used in the treatment of genital ulcers associated with Behçet's syndrome.

In some embodiments, the compound of formula (I), a stereoisomer thereof, or a pharmaceutically acceptable salt thereof of the present disclosure has good compliance when used in the treatment of genital ulcers associated with Behçet's syndrome.

In some embodiments, the compound of formula (I), a stereoisomer thereof, or a pharmaceutically acceptable salt thereof of the present disclosure does not require titrated dosing when used in the treatment of genital ulcers associated with Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of genital ulcers associated with Behçet's syndrome.

In some embodiments, the compound of formula (I), a stereoisomer thereof, or a pharmaceutically acceptable salt thereof of the present disclosure has excellent therapeutic effects when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of genital ulcers associated with Behçet's syndrome.

In some embodiments, the compound of formula (I), a stereoisomer thereof, or a pharmaceutically acceptable salt thereof of the present disclosure can be used in the treatment of genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of genital ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of genital ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of genital ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of genital ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of genital ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of genital ulcers associated with Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of genital ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of genital ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of genital ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of genital ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good tolerability when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good compliance when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not require titrated dosing when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has a rapid onset of action when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has favorable safety when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good tolerability when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good compliance when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not require titrated dosing when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has a rapid onset of action when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can be used in the treatment of Behçet's syndrome, oral ulcers associated with Behçet's syndrome, or genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject having a medical history of pulmonary tuberculosis infection, tuberculosis, and/or tuberculous pleurisy, oral ulcers associated with Behçet's syndrome in a subject suffering from latent tuberculosis infection, or genital ulcers associated with Behçet's syndrome in a subject suffering from latent tuberculosis infection, and pulmonary tuberculosis infection, tuberculosis, and/or tuberculous pleurisy as well as latent tuberculosis infection is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can reduce the area under the curve of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can reduce the number of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can alleviate oral ulcer pain when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve mucocutaneous involvement in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve joint involvement in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not diminish immunity in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In still another aspect, the present disclosure relates to the use of a compound of formula (I), a stereoisomer thereof, or a pharmaceutically acceptable salt thereof in the preparation of a medicament for the treatment of Behçet's syndrome in a subject,

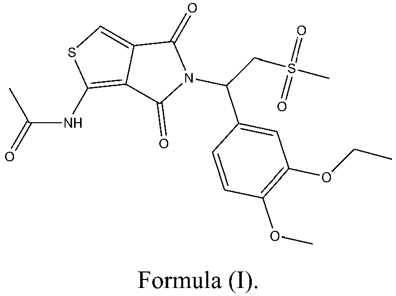

In still another aspect, the present disclosure relates to the use of a compound of formula (I), a stereoisomer thereof, or a pharmaceutically acceptable salt thereof in the preparation of a medicament for the treatment of oral ulcers associated with Behçet's syndrome in a subject,

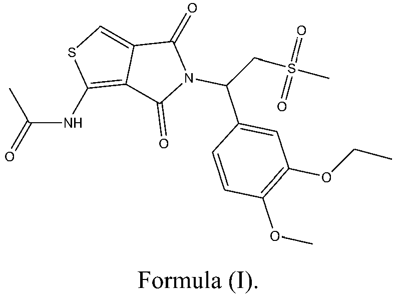

In still another aspect, the present disclosure relates to the use of a compound of formula (I), a stereoisomer thereof, or a pharmaceutically acceptable salt thereof in the preparation of a medicament for the treatment of genital ulcers associated with Behçet's syndrome in a subject,

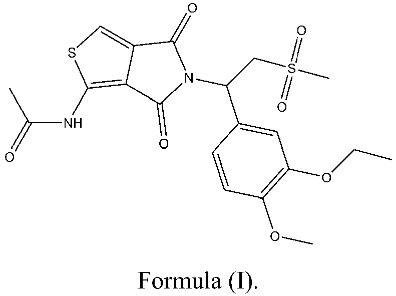

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg or 180 mg.

In some embodiments, the compound of formula (I), a stereoisomer thereof, or a pharmaceutically acceptable salt thereof is administered to the subject daily at a dose of 60 mg to 150 mg.

In some embodiments, the compound of formula (I), a stereoisomer thereof, or a pharmaceutically acceptable salt thereof is administered to the subject daily at a dose of 90 mg to 150 mg.

In some embodiments, the compound of formula (I), a stereoisomer thereof, or a pharmaceutically acceptable salt thereof is administered to the subject daily at a dose of 30 mg, 45 mg, 60 mg, 90 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof is administered to the subject twice daily at a dose of 45 mg to 60 mg each time.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg and 150 mg.

In some embodiments, the compound of formula (I), a stereoisomer thereof, or a pharmaceutically acceptable salt thereof has a unit dosage of 15 mg to 75 mg.

In some embodiments, the compound of formula (I), a stereoisomer thereof, or a pharmaceutically acceptable salt thereof has a unit dosage of 15 mg to 150 mg.

In some embodiments, an exemplary unit dosage of the compound of formula (I), a stereoisomer thereof, or a pharmaceutically acceptable salt thereof of the present disclosure includes, but is not limited to, 15 mg, 30 mg, 45 mg, 60 mg and 75 mg.

In some embodiments, the compound of formula (I), a stereoisomer thereof, or a pharmaceutically acceptable salt thereof has a unit dosage of 15 mg, 30 mg, 45 mg, 60 mg, 75 mg, 90 mg, 120 mg, and 150 mg.

In some embodiments, the compound of formula (I), a stereoisomer thereof, or a pharmaceutically acceptable salt thereof is administered to the subject in a tablet or capsule form.

In some embodiments, the compound of formula (I), a stereoisomer thereof, or a pharmaceutically acceptable salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse, and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the compound of formula (I), a stereoisomer thereof, or a pharmaceutically acceptable salt thereof of the present disclosure has good tolerability when used in the treatment of Behçet's syndrome.

In some embodiments, the compound of formula (I), a stereoisomer thereof, or a pharmaceutically acceptable salt thereof of the present disclosure has good compliance when used in the treatment of Behçet's syndrome.

In some embodiments, the compound of formula (I), a stereoisomer thereof, or a pharmaceutically acceptable salt thereof of the present disclosure does not require titrated dosing when used in the treatment of Behçet's syndrome.

In some embodiments, the compound of formula (I), a stereoisomer thereof, or a pharmaceutically acceptable salt thereof of the present disclosure has a rapid onset of action when used in the treatment of Behçet's syndrome.

In some embodiments, the compound of formula (I), a stereoisomer thereof, or a pharmaceutically acceptable salt thereof of the present disclosure has excellent therapeutic effects when used in the treatment of Behçet's syndrome.

In some embodiments, the compound of formula (I), a stereoisomer thereof, or a pharmaceutically acceptable salt thereof of the present disclosure has favorable safety when used in the treatment of Behçet's syndrome.

In some embodiments, the compound of formula (I), a stereoisomer thereof, or a pharmaceutically acceptable salt thereof of the present disclosure can be used in the treatment of Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome.

In some embodiments, the compound of formula (I), a stereoisomer thereof, or a pharmaceutically acceptable salt thereof of the present disclosure can reduce the area under the curve of oral ulcers when used in the treatment of Behçet's syndrome.

In some embodiments, the compound of formula (I), a stereoisomer thereof, or a pharmaceutically acceptable salt thereof of the present disclosure can reduce the number of oral ulcers when used in the treatment of Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome.

In some embodiments, the compound of formula (I), a stereoisomer thereof, or a pharmaceutically acceptable salt thereof of the present disclosure can alleviate oral ulcer pain when used in the treatment of Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome.

In some embodiments, the compound of formula (I), a stereoisomer thereof, or a pharmaceutically acceptable salt thereof of the present disclosure can improve mucocutaneous involvement in the subject when used in the treatment of Behçet's syndrome.

In some embodiments, the compound of formula (I), a stereoisomer thereof, or a pharmaceutically acceptable salt thereof of the present disclosure can improve joint involvement in the subject when used in the treatment of Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome.

In some embodiments, the compound of formula (I), a stereoisomer thereof, or a pharmaceutically acceptable salt thereof of the present disclosure does not diminish immunity in the subject when used in the treatment of Behçet's syndrome.

In some embodiments, the compound of formula (I), a stereoisomer thereof, or a pharmaceutically acceptable salt thereof of the present disclosure has good tolerability when used in the treatment of oral ulcers associated with Behçet's syndrome.

In some embodiments, the compound of formula (I), a stereoisomer thereof, or a pharmaceutically acceptable salt thereof of the present disclosure has good compliance when used in the treatment of oral ulcers associated with Behçet's syndrome.

In some embodiments, the compound of formula (I), a stereoisomer thereof, or a pharmaceutically acceptable salt thereof of the present disclosure does not require titrated dosing when used in the treatment of oral ulcers associated with Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of oral ulcers associated with Behçet's syndrome.

In some embodiments, the compound of formula (I), a stereoisomer thereof, or a pharmaceutically acceptable salt thereof of the present disclosure has excellent therapeutic effects when used in the treatment of oral ulcers associated with Behçet's syndrome.

In some embodiments, the compound of formula (I), a stereoisomer thereof, or a pharmaceutically acceptable salt thereof of the present disclosure has favorable safety when used in the treatment of oral ulcers associated with Behçet's syndrome.

In some embodiments, the compound of formula (I), a stereoisomer thereof, or a pharmaceutically acceptable salt thereof of the present disclosure can be used in the treatment of oral ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of oral ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of oral ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of oral ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of oral ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of oral ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of oral ulcers associated with Behçet's syndrome.

In some embodiments, the compound of formula (I), a stereoisomer thereof, or a pharmaceutically acceptable salt thereof of the present disclosure can improve related genital ulcers in the subject, and/or alleviate genital ulcer pain when used in the treatment of oral ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of oral ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of oral ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of oral ulcers associated with Behçet's syndrome.

In some embodiments, the compound of formula (I), a stereoisomer thereof, or a pharmaceutically acceptable salt thereof of the present disclosure does not diminish immunity in the subject in the treatment of oral ulcers associated with Behçet's syndrome.

In some embodiments, the compound of formula (I), a stereoisomer thereof, or a pharmaceutically acceptable salt thereof of the present disclosure has good tolerability when used in the treatment of genital ulcers associated with Behçet's syndrome.

In some embodiments, the compound of formula (I), a stereoisomer thereof, or a pharmaceutically acceptable salt thereof of the present disclosure has good compliance when used in the treatment of genital ulcers associated with Behçet's syndrome.

In some embodiments, the compound of formula (I), a stereoisomer thereof, or a pharmaceutically acceptable salt thereof of the present disclosure does not require titrated dosing when used in the treatment of genital ulcers associated with Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of genital ulcers associated with Behçet's syndrome.

In some embodiments, the compound of formula (I), a stereoisomer thereof, or a pharmaceutically acceptable salt thereof of the present disclosure has excellent therapeutic effects when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good tolerability when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good compliance when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not require titrated dosing when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has a rapid onset of action when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has excellent therapeutic effects when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has favorable safety when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can be used in the treatment of genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of genital ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of genital ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of genital ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of genital ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of genital ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of genital ulcers associated with Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of genital ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of genital ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of genital ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of genital ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of genital ulcers associated with Behçet's syndrome.

In yet another aspect, the present disclosure relates to a pharmaceutical composition for use in the treatment of Behçet's syndrome in a subject, comprising a compound of formula (I), a stereoisomer thereof, or a pharmaceutically acceptable salt thereof, and a pharmaceutically acceptable carrier, diluent or excipient,

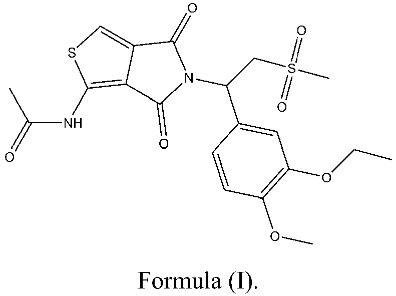

In yet another aspect, the present disclosure relates to a pharmaceutical composition for use in the treatment of oral ulcers associated with Behçet's syndrome in a subject, comprising a compound of formula (I), a stereoisomer thereof, or a pharmaceutically acceptable salt thereof, and a pharmaceutically acceptable carrier, diluent or excipient,

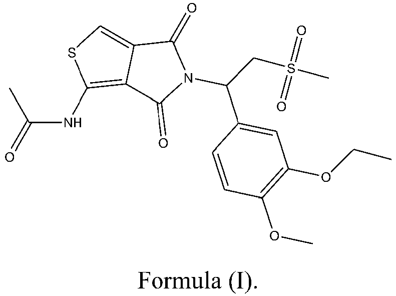

In yet another aspect, the present disclosure relates to a pharmaceutical composition for use in the treatment of genital ulcers associated with Behçet's syndrome in a subject, comprising a compound of formula (I), a stereoisomer thereof, or a pharmaceutically acceptable salt thereof, and a pharmaceutically acceptable carrier, diluent or excipient,

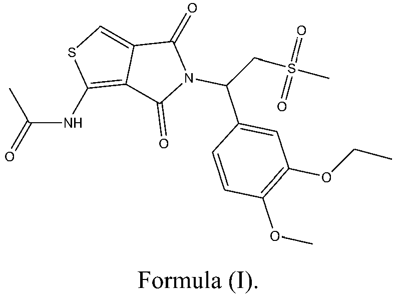

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg or 180 mg.

In some embodiments, the compound of formula (I), a stereoisomer thereof, or a pharmaceutically acceptable salt thereof is administered to the subject daily at a dose of 60 mg to 150 mg.

In some embodiments, the compound of formula (I), a stereoisomer thereof, or a pharmaceutically acceptable salt thereof is administered to the subject daily at a dose of 90 mg to 150 mg.

In some embodiments, the compound of formula (I), a stereoisomer thereof, or a pharmaceutically acceptable salt thereof is administered to the subject daily at a dose of 30 mg, 45 mg, 60 mg, 90 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof is administered to the subject twice daily at a dose of 45 mg to 60 mg each time.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg and 150 mg.

In some embodiments, the compound of formula (I), a stereoisomer thereof, or a pharmaceutically acceptable salt thereof has a unit dosage of 15 mg to 75 mg.

In some embodiments, the compound of formula (I), a stereoisomer thereof, or a pharmaceutically acceptable salt thereof has a unit dosage of 15 mg to 150 mg.

In some embodiments, an exemplary unit dosage of the compound of formula (I), a stereoisomer thereof, or a pharmaceutically acceptable salt thereof of the present disclosure includes, but is not limited to, 15 mg, 30 mg, 45 mg, 60 mg and 75 mg.

In some embodiments, the compound of formula (I), a stereoisomer thereof, or a pharmaceutically acceptable salt thereof has a unit dosage of 15 mg, 30 mg, 45 mg, 60 mg, 75 mg, 120 mg, and 150 mg.

In some embodiments, the compound of formula (I), a stereoisomer thereof, or a pharmaceutically acceptable salt thereof is administered to the subject in a tablet or capsule form.

In some embodiments, the compound of formula (I), a stereoisomer thereof, or a pharmaceutically acceptable salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse, and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the compound of formula (I), a stereoisomer thereof, or a pharmaceutically acceptable salt thereof of the present disclosure has good tolerability when used in the treatment of Behçet's syndrome.

In some embodiments, the compound of formula (I), a stereoisomer thereof, or a pharmaceutically acceptable salt thereof of the present disclosure has good compliance when used in the treatment of Behçet's syndrome.

In some embodiments, the compound of formula (I), a stereoisomer thereof, or a pharmaceutically acceptable salt thereof of the present disclosure does not require titrated dosing when used in the treatment of Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of Behçet's syndrome.

In some embodiments, the compound of formula (I), a stereoisomer thereof, or a pharmaceutically acceptable salt thereof of the present disclosure has excellent therapeutic effects when used in the treatment of Behçet's syndrome.

In some embodiments, the compound of formula (I), a stereoisomer thereof, or a pharmaceutically acceptable salt thereof of the present disclosure has favorable safety when used in the treatment of Behçet's syndrome.

In some embodiments, the compound of formula (I), a stereoisomer thereof, or a pharmaceutically acceptable salt thereof of the present disclosure can be used in the treatment of Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome.

In some embodiments, the compound of formula (I), a stereoisomer thereof, or a pharmaceutically acceptable salt thereof of the present disclosure can reduce the area under the curve of oral ulcers when used in the treatment of Behçet's syndrome.

In some embodiments, the compound of formula (I), a stereoisomer thereof, or a pharmaceutically acceptable salt thereof of the present disclosure can reduce the number of oral ulcers when used in the treatment of Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome.

In some embodiments, the compound of formula (I), a stereoisomer thereof, or a pharmaceutically acceptable salt thereof of the present disclosure can alleviate oral ulcer pain when used in the treatment of Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome.

In some embodiments, the compound of formula (I), a stereoisomer thereof, or a pharmaceutically acceptable salt thereof of the present disclosure can improve mucocutaneous involvement in the subject when used in the treatment of Behçet's syndrome.

In some embodiments, the compound of formula (I), a stereoisomer thereof, or a pharmaceutically acceptable salt thereof of the present disclosure can improve joint involvement in the subject when used in the treatment of Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome.

In some embodiments, the compound of formula (I), a stereoisomer thereof, or a pharmaceutically acceptable salt thereof of the present disclosure does not diminish immunity in the subject when used in the treatment of Behçet's syndrome.

In some embodiments, the compound of formula (I), a stereoisomer thereof, or a pharmaceutically acceptable salt thereof of the present disclosure has good tolerability when used in the treatment of oral ulcers associated with Behçet's syndrome.

In some embodiments, the compound of formula (I), a stereoisomer thereof, or a pharmaceutically acceptable salt thereof of the present disclosure has good compliance when used in the treatment of oral ulcers associated with Behçet's syndrome.

In some embodiments, the compound of formula (I), a stereoisomer thereof, or a pharmaceutically acceptable salt thereof of the present disclosure does not require titrated dosing when used in the treatment of oral ulcers associated with Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of oral ulcers associated with Behçet's syndrome.

In some embodiments, the compound of formula (I), a stereoisomer thereof, or a pharmaceutically acceptable salt thereof of the present disclosure has excellent therapeutic effects when used in the treatment of oral ulcers associated with Behçet's syndrome.

In some embodiments, the compound of formula (I), a stereoisomer thereof, or a pharmaceutically acceptable salt thereof of the present disclosure has favorable safety when used in the treatment of oral ulcers associated with Behçet's syndrome.

In some embodiments, the compound of formula (I), a stereoisomer thereof, or a pharmaceutically acceptable salt thereof of the present disclosure can be used in the treatment of oral ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of oral ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of oral ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of oral ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of oral ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of oral ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of oral ulcers associated with Behçet's syndrome.

In some embodiments, the compound of formula (I), a stereoisomer thereof, or a pharmaceutically acceptable salt thereof of the present disclosure can improve related genital ulcers in the subject, and/or alleviate genital ulcer pain when used in the treatment of oral ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of oral ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of oral ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of oral ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of oral ulcers associated with Behçet's syndrome.

In some embodiments, the compound of formula (I), a stereoisomer thereof, or a pharmaceutically acceptable salt thereof of the present disclosure has good tolerability when used in the treatment of genital ulcers associated with Behçet's syndrome.

In some embodiments, the compound of formula (I), a stereoisomer thereof, or a pharmaceutically acceptable salt thereof of the present disclosure has good compliance when used in the treatment of genital ulcers associated with Behçet's syndrome.

In some embodiments, the compound of formula (I), a stereoisomer thereof, or a pharmaceutically acceptable salt thereof of the present disclosure does not require titrated dosing when used in the treatment of genital ulcers associated with Behçet's syndrome.

In some embodiments, the compound of formula (I), a stereoisomer thereof, or a pharmaceutically acceptable salt thereof of the present disclosure can be administered to the subject for a short or long term without inducing depression in the subject.

In some embodiments, the compound of formula (I), a stereoisomer thereof, or a pharmaceutically acceptable salt thereof of the present disclosure has a rapid onset of action when used in the treatment of genital ulcers associated with Behçet's syndrome.

In some embodiments, the compound of formula (I), a stereoisomer thereof, or a pharmaceutically acceptable salt thereof of the present disclosure has excellent therapeutic effects when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of genital ulcers associated with Behçet's syndrome.

In some embodiments, the compound of formula (I), a stereoisomer thereof, or a pharmaceutically acceptable salt thereof of the present disclosure has favorable safety when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can be used in the treatment of genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of genital ulcers associated with Behçet's syndrome in a subject with a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of genital ulcers associated with Behçet's syndrome.

In some embodiments, the compound of formula (I), a stereoisomer thereof, or a pharmaceutically acceptable salt thereof of the present disclosure can reduce the area under the curve of oral ulcers when used in the treatment of genital ulcers associated with Behçet's syndrome.

In some embodiments, the compound of formula (I), a stereoisomer thereof, or a pharmaceutically acceptable salt thereof of the present disclosure can reduce the number of oral ulcers when used in the treatment of genital ulcers associated with Behçet's syndrome.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of genital ulcers associated with Behçet's syndrome.

In some embodiments, the compound of formula (I), a stereoisomer thereof, or a pharmaceutically acceptable salt thereof of the present disclosure can alleviate oral ulcer pain when used in the treatment of genital ulcers associated with Behçet's syndrome.

In some embodiments, the compound of formula (I), a stereoisomer thereof, or a pharmaceutically acceptable salt thereof of the present disclosure can improve related genital ulcers, and/or alleviate genital ulcer pain when used in the treatment of genital ulcers associated with Behçet's syndrome.

In some embodiments, the compound of formula (I), a stereoisomer thereof, or a pharmaceutically acceptable salt thereof of the present disclosure can improve mucocutaneous involvement in the subject when used in the treatment of genital ulcers associated with Behçet's syndrome.

In some embodiments, the compound of formula (I), a stereoisomer thereof, or a pharmaceutically acceptable salt thereof of the present disclosure can improve joint involvement in the subject when used in the treatment of genital ulcers associated with Behçet's syndrome.

In some embodiments, the compound of formula (I), a stereoisomer thereof, or a pharmaceutically acceptable salt thereof of the present disclosure does not induce or exacerbate anxiety or depression in the subject when used in the treatment of genital ulcers associated with Behçet's syndrome.

In some embodiments, the compound of formula (I), a stereoisomer thereof, or a pharmaceutically acceptable salt thereof of the present disclosure does not diminish immunity in the subject when used in the treatment of genital ulcers associated with Behçet's syndrome.

In some embodiments, the compound of formula (I), a stereoisomer thereof, or a pharmaceutically acceptable salt thereof of the present disclosure has good tolerability when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has good compliance when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not require titrated dosing when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure has a rapid onset of action when used in the treatment of Behçet's syndrome in a subject suffering from latent tuberculosis infection, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can be used in the treatment of Behçet's syndrome, oral ulcers associated with Behçet's syndrome, or genital ulcers associated with Behçet's syndrome in a subject suffering from latent tuberculosis infection, and the latent tuberculosis infection is not reactivated after treatment.

In some embodiments, the compound of formula (I), a stereoisomer thereof, or a pharmaceutically acceptable salt thereof of the present disclosure can be used in the treatment of Behçet's syndrome in a subject having a medical history of pulmonary tuberculosis infection, tuberculosis, and/or tuberculous pleurisy, oral ulcers associated with Behçet's syndrome in a subject suffering from latent tuberculosis infection, or genital ulcers associated with Behçet's syndrome in a subject suffering from latent tuberculosis infection, and pulmonary tuberculosis infection, tuberculosis, and/or tuberculous pleurisy as well as latent tuberculosis infection is/are not reactivated after treatment.

In some embodiments, the compound of formula (I), a stereoisomer thereof, or a pharmaceutically acceptable salt thereof of the present disclosure can improve mucocutaneous manifestations of oral ulcers in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can reduce the area under the curve of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can reduce the number of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can shorten the time required for oral ulcer healing, and/or reduce the recurrence of oral ulcers, and/or prolong the duration of complete remission of oral ulcers when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can alleviate oral ulcer pain when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve related genital ulcers, and/or alleviate genital ulcer pain when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve mucocutaneous involvement in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure can improve joint involvement in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not induce or exacerbate anxiety or depression in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

In some embodiments, the compound of formula (I), a stereoisomer thereof, or a pharmaceutically acceptable salt thereof of the present disclosure does not diminish immunity in the subject when used in the treatment of Behçet's syndrome, oral ulcers associated with Behçet's syndrome in a subject suffering from latent tuberculosis infection, or genital ulcers associated with Behçet's syndrome in a subject suffering from latent tuberculosis infection.

The present disclosure will be illustrated in detail through the following examples in order to better understand various aspects and advantages of the present disclosure. However, it should be understood that the following examples are non-limiting and merely used to illustrate certain embodiments of the present disclosure.

### Examples

Glossary:
BID: twice daily
VAS: Visual Analogue Scale Score for oral ulcer pain
PGA: Physician's Global Assessment
BVAS: Birmingham Vasculitis Activity Score
Ritchie: Articular Index Score

### Example 1

### Preparation of compound of formula (I)

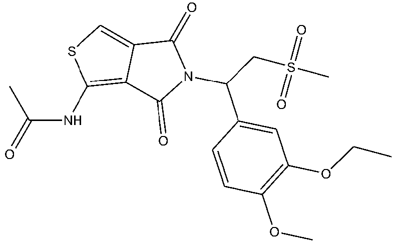

N-[5-[1-(3-ethoxy-4-methoxyphenyl)-2-(methylsulfonyl)ethyl]-4,6-dioxo-5,6-dihydro-4H-thieno[3,4-c]pyrrol-1-yl]acetamide was prepared with reference to the preparation method 2 of Example 3 in CN101885731A.

### Example 2

### Preparation of stereoisomer of compound of formula (I)

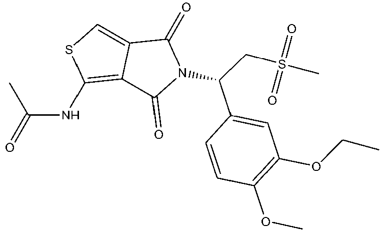

(S)-N-[5-[1-(3-ethoxy-4-methoxyphenyl)-2-(methylsulfonyl)ethyl]-4,6-dioxo-5,6-dihydro-4H-thieno[3,4-c]pyrrol-1-yl]acetamide was prepared with reference to the preparation method of Example 1 in CN116332954A.

### Example 3

### Clinical Study in Patients

In this study, 90 patients with Behçet's syndrome (aged 18 to 75 years inclusive), which were diagnosed with Behçet's disease in accordance with the International Diagnostic (Classification) Criteria for Behçet's Disease established in 2013 (ICBD-2013) by the International Study Group, were enrolled and randomly assigned at a ratio of 2:2:1:1 to one of the following groups: the high-dose group of Example 2 (60 mg BID group), the low-dose group of Example 2 (45 mg BID group), the placebo (core treatment period) + 60 mg BID (extension treatment period) group, or the placebo (core treatment period) + 45 mg BID (extension treatment period) group. During the core treatment period, all subjects were administered twice daily for 12 consecutive weeks. Subjects in the high-dose group and the low-dose group during the core treatment period continued to receive the same dosages for an additional 12 weeks during the extension period. Subjects in the placebo group during the core treatment period were switched to either the high-dose group or the low-dose group and received treatment for an additional 12 weeks during the extension period. Both subjects and investigators remained blinded throughout this phase. Withdrawal observation period: all subjects in this study (including those who discontinued treatment prematurely for any reason) were required to be observed for 4 weeks following the last administration.

Primary efficacy endpoint: Area under the curve (AUC) of the number of oral ulcers in BD patients from baseline to Week 12 of treatment.

Secondary efficacy endpoints: Least squares mean (standard error/95% CI) of the difference in AUC between the test drug and the placebo; mean number of oral ulcers at Week 12 (standard deviation); and complete remission rate at Week 12 (n/m, %).

**Table 1. Efficacy assessment for 12-week core treatment period**

| Area under the curve (AUC¹) of the number of oral ulcers in BD patients from baseline to Week 12 of treatment (FAS) | | | |
|---|---|---|---|
| | 45mg BID (N=26) | 60mg BID (N=27) | Placebo total (N=26) |
| | | | |
| Least squares mean (standard error) | 108.3 (20.45) | 77.3 (19.70) | 207.5 (20.07) |
| 95% Confidence Interval | (67.57, 149.09) | (38.08, 116.61) | (167.47, 247.46) |
| | | | |
| Comparison among groups | | | |
| 60 mg group VS placebo group | | | |
| P value | | <0.0001 | |
| | | | |
| 45 mg group VS placebo group | | | |
| P value | 0.0004 | | |
| | | | |
| (60 mg+45 mg) group VS placebo group | | | |
| P value | | | <0.0001 |

**Table 2. Efficacy assessment for 12-week core treatment period**

| Complete remission rate of oral ulcers (proportion of patients free of oral ulcers) at Day 3, Day 7 and Week 12 of treatment compared with baseline (FAS) | | | |
|---|---|---|---|
| | 45mg BID (N=26) | 60 mg BID (N=27) | Placebo total (N=26) |
| Day 3 | | | |
| complete remission, n/m, (%)¹ | 5/25 (20.0) | 2/27 (7.4) | 0 |
| | | | |

| Day 7 | | | |
|---|---|---|---|
| complete remission, n/m, (%)¹ | 8/24 (33.3) | 8/26 (30.8) | 1/25 (4.0) |
| | | | |

| Week 12 | | | |
|---|---|---|---|
| complete remission, n/m, (%)¹ | 10/17 (58.8) | 12/17 (70.6) | 5/17 (29.4) |
| n: number of subjects with complete remission of oral ulcers; | | | |
| m: number of subjects with non-missing information on oral ulcers during the corresponding visit. | | | |

Results of this clinical study:
1. Efficacy
   Primary efficacy endpoint: Compared with the placebo group, the AUC of the number of oral ulcers from Week 0 to Week 12 was significantly reduced in both the 45 mg BID and 60 mg BID dose groups, with statistical significance. Both treatment groups showed statistically significant differences compared with the placebo group. Compared with the placebo group, the 45 mg BID and 60 mg BID groups showed a decrease in the area under the curve (AUC) of the number of oral ulcers from Week 0 to Week 12 by 99.1 and 130.1, respectively, and complete remission rates of oral ulcers at Week 12 of 58.8% and 70.6%, respectively.
2. Rapid onset of clinical efficacy in the treatment of oral ulcers associated with Behçet's disease
   At Day 3 of treatment, preliminary efficacy was observed compared with the placebo group.
   At Day 7 of treatment, more than 30% of subjects in the active drug treatment groups achieved complete remission of oral ulcers, and this efficacy remained stable throughout the treatment period.
3. Safety
   The overall safety and tolerability were favorable.

All adverse reactions were mild or moderate, most occurring at the initial stage of drug treatment, and most resolved spontaneously within one week without clinical intervention. No severe adverse reactions occurred during the trial.

### Example 4

### Clinical Study in Patients

In this study, 90 patients with Behçet's syndrome (aged 18 to 75 years inclusive), which were diagnosed with Behçet's disease in accordance with the International Diagnostic (Classification) Criteria for Behçet's Disease established in 2013 (ICBD-2013) by the International Study Group, were enrolled and randomly assigned at a ratio of 2:2:1:1 to one of the following groups: the high-dose group of Example 2 (60 mg BID group), the low-dose group of Example 2 (45 mg BID group), the placebo (core treatment period) + 60 mg BID (extension treatment period) group, or the placebo (core treatment period) + 45 mg BID (extension treatment period) group. During the core treatment period, all subjects were administered twice daily for 12 consecutive weeks. Subjects in the high-dose group and the low-dose group during the core treatment period continued to receive the same dosages for an additional 12 weeks during the extension period. Subjects in the placebo group during the core treatment period were switched to either the high-dose group or the low-dose group and received treatment for an additional 12 weeks during the extension period. Both subjects and investigators remained blinded throughout this phase. Withdrawal observation period: all subjects in this study (including those who discontinued treatment prematurely for any reason) were required to be observed for 4 weeks following the last administration.

Inclusion Criteria:
1. Have understood and voluntarily signed the informed consent form for this study;
2. Aged 18 to 75 years (inclusive), with no gender restriction.
3. Have a confirmed diagnosis of Behcet's disease (BD) in accordance with the International Diagnostic (Classification) Criteria for Behçet's Disease (ICBD-2013).
4. Have at least two oral ulcers at Visit 1 (screening period), and
   a. if Visit 2 occurs at least 14 days after Visit 1, the subject has at least two oral ulcers at Visit 2 (day of randomization); or
   b. if Visit 2 occurs within 0 to 42 days after Visit 1, the subject has at least three oral ulcers at Visit 2 (day of randomization);
5. Require systemic treatment for oral ulcers: depending on the disease severity and extent of involved regions, it is judged by the investigators that the subject's oral ulcers are not suitable for topical treatment, or that the subject's oral ulcers cannot be effectively controlled by topical treatment, thus requiring systemic treatment.
6. Throughout the entire study from signing the informed consent form to 3 months after the last administration, female subjects of childbearing potential and male subjects who have not undergone vasectomy must use effective contraceptive measures (effective contraceptive measures include: vasectomy, abstinence, intrauterine device (IUD), hormonal contraceptives (oral, patch, ring, injection, implant) and barrier methods (diaphragm, cervical cap, sponge, condom).
7. Be able to comply with the follow-up schedule and other protocol requirements.

Primary efficacy endpoint:
Area under the curve (AUC) of the number of oral ulcers in BD patients from baseline to Week 12 of treatment.

Secondary efficacy endpoints:
Proportion of subjects with complete remission of oral ulcers (healing of oral ulcers) at Week 6 and maintenance of such complete remission for 6 weeks;
Complete remission rate of oral ulcers at week 12 compared with the placebo group;
Change from baseline in the visual analogue scale (VAS) score for oral ulcer pain at Week 12 of treatment;
Change from baseline in the number of oral ulcers at Week 12 of treatment;
Time to oral ulcer healing (complete remission) during the core treatment period, defined as the time to the first occurrence of complete remission of oral ulcers in a subject; ulcer healing is defined as complete flattening of ulcer surface, absence of congestion, and absence of pain;
Proportion of subjects free of oral ulcers who achieved initial complete remission and maintained complete remission during the core treatment period;
Number of recurrent oral ulcers after complete remission in subjects during the core treatment period, i.e., the number of oral ulcers at the first recurrence after complete remission in subjects;
Change in physician's global assessment at Week 12 in BD patients with skin lesions (acneiform lesions, folliculitis, and erythema nodosum) at baseline during the core treatment period; and
Change from baseline in complete remission rate of genital ulcers (proportion of subjects free of genital ulcers) at Week 12 of treatment.

Results of this clinical study:
1. Efficacy:

**Table 3. Efficacy assessment data for core treatment period**

| | 45 mg (N=29) | 60 mg (N=31) | Placebo (N=30) |
|---|---|---|---|
| Area under the curve (AUC) of the number of oral ulcers, mean ± SD | 103.9±18.8 | 66.1±17.7 | 208.7±18.1 |
| Difference in least squares mean (95% CI) for AUC of the number of oral ulcers | -104.8 (-156.3, -53.2) | -142.5 (-192.4, -92.7) | |
| P value | <0.0001 | <0.0001 | |
| Complete remission rate of oral ulcers at Week 12, % | 58.8 | 70.6 | |
| Change from baseline in VAS score, mean ± SD | -42.2±4.7 | -40.5±4.9 | -28.5±4.6 |
| Difference in least squares mean (95%CI) for change from baseline in VAS score | -13.6 (-26.6, -0.7) | -12.0 (-25.5, 1.5) | |
| P value | 0.0199 | 0.0404 | |
| Change in the number of oral ulcers, mean ± SD | -2.8±0.3 | -2.9±0.3 | -1.6±0.3 |
| Difference in least squares mean (95% CI) | -1.2 (-2.0, -0.4) | -1.3 (-2.1, -0.4) | |
| P value | 0.0026 | 0.0016 | |
| Proportion of subjects achieving complete remission of oral ulcers (no oral ulcer) at Week 6 of treatment and maintaining complete remission for consecutive 6 weeks, % | 27.6 | 38.7 | 3.3 |
| Difference in least squares mean (95%CI) | 23.7 (5.8, 41.6) | 35.5 (17.5, 53.6) | |
| P value | 0.0047 | <0.0001 | |
| Complete remission rate of oral ulcers at Week 12 of treatment, % | 37.9 | 45.2 | 16.7 |
| Difference in least squares mean (95% CI) | 20.0 (-2.4, 42.3) | 28.7 (6.4, 51.0) | |
| P value | 0.0401 | 0.0058 | |
| Proportion of subjects free of oral ulcers who achieved initial complete remission and maintained complete remission, % | 34.5 | 51.6 | 13.3 |
| Difference in least squares mean (95% CI) | 20.0 (-1.6, 41.7) | 38.4 (16.5, 60.2) | |
| P value | 0.0346 | 0.0003 | |
| Time to oral ulcer healing (days) (95% CI) | 16 (8.0, 46.0) | 15 (9.0, 29.0) | NA (70.0, NA) |
| P value | 0.0002 | <0.0001 | |

The proportions of subjects achieving complete remission of oral ulcers (no oral ulcers) at Week 6 of treatment and maintaining such complete remission for consecutive 6 weeks were 27.6% in the 45 mg group, 38.7% in the 60 mg group, and 3.3% in the placebo group, respectively. The differences between the 45 mg group and the placebo group and between the 60 mg group and the placebo group were 23.7% and 35.5%, respectively.

At Week 12 of treatment, the decreases in the number of ulcers from baseline were 2.7, 2.9 and 1.7 in the 45 mg group, 60 mg group and placebo group, respectively. Statistically significant differences in the change from baseline in the number of oral ulcers were observed in both the 60 mg group vs. placebo group (-1.3, *p* = 0.0016) and the 45 mg group vs. placebo group (-1.2, *p* = 0.00026).

The median time to oral ulcer healing was 16 days, with 95% C.I. of (8, 46) in the 60 mg group; and 15 days, with 95% C.I. of (9, 29) in the 45 mg group. The median healing time in the placebo group was not estimable. Statistically significant differences in time to oral ulcer healing were observed for both the 45 mg group and 60 mg group compared with the placebo group during the core treatment period.

The proportions of subjects achieving first complete remission of oral ulcers and maintaining complete remission during the core treatment period were 34.5% in the 45 mg group, 51.6% in the 60 mg group, and 13.3% in the placebo group, respectively.

At Week 12, 2 subjects in the 45 mg dose group who had genital ulcers at baseline and remained in this study achieved complete remission of genital ulcers. At Week 12, 2 subjects in the 60 mg dose group who had genital ulcers at baseline and remained in this study achieved complete remission of genital ulcers.

At Week 12, 2 out of 3 patients in the 60 mg dose group who had joint tenderness at baseline and remained in this study had a reduction in the number of tender joints to 0.

At Week 12, among 9 patients in the 45 mg dose group who had skin lesions at baseline per skin PGA assessment and remained in this study, 5 patients achieved a PGA score of 0. Specifically, 1 patient with acneiform lesions at baseline per PGA had complete resolution of acneiform lesions at Week 12; 2 patients with erythema nodosum at baseline per PGA had complete resolution of erythema nodosum at Week 12; 1 patient with folliculitis at baseline per PGA had complete resolution of folliculitis at Week 12; and 1 patient with acnes, folliculitis and erythema nodosum at baseline per PGA had complete resolution of all lesions at Week 12. At Week 12, among 8 patients in the 60 mg dose group who had skin lesions at baseline per skin PGA assessment and remained in this study, 5 patients achieved a PGA score of 0. Specifically, 2 patients with acnes at baseline per PGA had complete resolution of acnes at Week 12; and 3 patients with folliculitis at baseline per PGA had complete resolution of folliculitis at Week 12.

### 2. Rapid onset of clinical efficacy in the treatment of oral ulcers associated with Behcet's disease

At Day 3 of treatment, preliminary efficacy was observed compared with the placebo group. At Day 7 of treatment, more than 30% of subjects in the active drug treatment groups achieved complete remission of oral ulcers (compared with 4% in the placebo group), and this efficacy remained stable throughout the treatment period.

### 3. Safety

The overall safety and tolerability were favorable.

All adverse reactions were mild or moderate. The most common adverse reactions were nausea, headache, diarrhea, upper respiratory tract infection, and vomiting. Most adverse reactions occurred at the initial stage of drug treatment, and resolved spontaneously within one week without clinical intervention.

No severe adverse reactions occurred up to the completion of the clinical study.

No deaths occurred up to the completion of the clinical study.

No adverse events of depression or anxiety were observed and no depression or suicidal tendency occurred up to the completion of the clinical study.

Three subjects reported a history related to tuberculosis, including tuberculosis (1 case) and pulmonary tuberculosis (2 cases). No cases of tuberculosis reactivation or new-onset tuberculosis were observed up to the completion of the clinical study.

### 4. Other results

Population pharmacokinetic and exposure-efficacy analysis demonstrated that the efficacy response rate increased with increasing dose.

### Example 5

### Clinical Study in Patients

A multicenter, randomized, double-blind, placebo-controlled, parallel-group phase III clinical trial evaluating the efficacy and safety in 305 subjects with moderate to severe chronic plaque psoriasis was conducted, among which 105 subjects were T-SPOT positive without active tuberculosis. Clinical observation and evaluation were conducted throughout a 16-week core treatment period, a 36-week extended treatment period, and a follow-up period (4 weeks). Anxiety and depression scales were used as the evaluation tools to observe and evaluate the anxiety and depression status of the subjects during the screening period, upon completion of the 16-week core period, and 4 weeks after the last administration.

The experimental results showed that, up to the completion of the clinical study, none of the 105 T-SPOT positive subjects without active tuberculosis, 7 subjects with confirmed prior pulmonary tuberculosis, 76 subjects with a history of tuberculosis, and 1 subject with prior tuberculous pleurisy experienced reactivation. No pulmonary tuberculosis-related clinical abnormalities were observed in chest X-ray or CT examination for all subjects.

**Table 4. Depression scale scores in a Phase III clinical trial of moderate-to-severe plaque psoriasis**

| | | Week 16 | | Week 56 | |
|---|---|---|---|---|---|
| | | Treatment group | Placebo group | Treatment group | Placebo group |
| Indicator | | (N=211) | (N=94) | (N=211) | (N=94) |
| HADS anxiety scores (points) | mean | 2.3 | 3.0 | 3.1 | 2.4 |
| HADS depression scores (points) | mean | 3.0 | 3.7 | 3.8 | 3.3 |

Up to the completion of the clinical study, there were no statistically significant differences (P>0.05) in the mean HADS anxiety scores and the mean HADS depression scores (± standard deviation) between the treatment group and placebo group. No adverse events of depression or anxiety associated with the study drug were observed in the subjects, and no depression or suicidal tendency occurred.

### Example 6

### Clinical Study in Patients

A Phase I clinical trial enrolling a total of 36 healthy Chinese subjects was conducted, with multiple oral administrations of the compound of Example 2 at doses of 15 mg BID, 30 mg BID, 60 mg BID and 75 mg BID, and placebo, respectively. The clinical trial showed favorable safety and good tolerability.

### Example 7

### Clinical Study in Patients

In a multicenter, randomized, double-blind, placebo-controlled, parallel-group Phase III clinical study evaluating the efficacy and safety in 78 subjects with Behçet's disease, including 1 subject with a confirmed diagnosis of previous pulmonary tuberculosis and 4 subjects with latent tuberculosis infection, no cases of tuberculosis reactivation occurred.

In the present disclosure, relational terms such as "first" and "second" are used merely to distinguish one entity or operation from another entity or operation, and do not necessarily require or imply any such actual relationship or order between these entities or operations.

It can be understood from the foregoing that although specific embodiments of the present disclosure have been described for illustrative purposes, various modifications or variations can be made by a person skilled in the art without departing from the spirit and scope of the present disclosure. All such modifications or variations should fall within the scope of the claims appended to the present disclosure.

## Claims

1. A method for treating Behçet's syndrome, comprising administering to a subject in need thereof a therapeutically effective amount of a compound of formula (I), a stereoisomer thereof, or a pharmaceutically acceptable salt thereof,

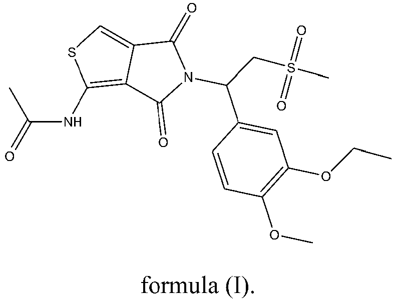

2. A method for treating oral ulcers associated with Behçet's syndrome, comprising administering to a subject in need thereof a therapeutically effective amount of a compound of formula (I), a stereoisomer thereof, or a pharmaceutically acceptable salt thereof,

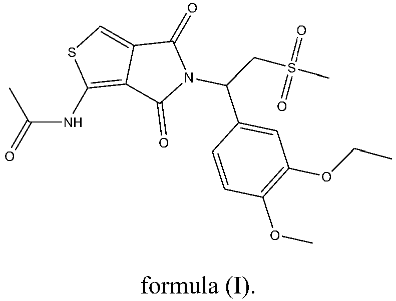

3. A method for treating genital ulcers associated with Behçet's syndrome, comprising administering to a subject in need thereof a therapeutically effective amount of a compound of formula (I), a stereoisomer thereof, or a pharmaceutically acceptable salt thereof,

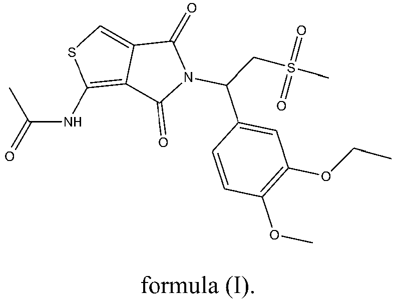

4. The method of any one of claims 1 to 3, wherein the compound of formula (I), a stereoisomer thereof, or a pharmaceutically acceptable salt thereof is administered to the subject daily at a dose of 30 mg to 180 mg.

5. The method of any one of claims 1 to 4, wherein the compound of formula (I), a stereoisomer thereof, or a pharmaceutically acceptable salt thereof is administered to the subject daily at a dose of 60 mg to 150 mg.

6. The method of any one of claims 1 to 5, wherein the compound of formula (I), a stereoisomer thereof, or a pharmaceutically acceptable salt thereof is administered to the subject daily at a dose of 150 mg.

7. The method of any one of claims 1 to 6, wherein the compound of formula (I), a stereoisomer thereof, or a pharmaceutically acceptable salt thereof is administered to the subject daily at a dose of 120 mg.

8. The method of any one of claims 1 to 7, wherein the compound of formula (I), a stereoisomer thereof, or a pharmaceutically acceptable salt thereof is administered to the subject at least once daily.

9. The method of any one of claims 1 to 8, wherein the compound of formula (I), a stereoisomer thereof, or a pharmaceutically acceptable salt thereof is administered to the subject at least twice daily.

10. The method of any one of claims 1 to 9, wherein the compound of formula (I), a stereoisomer thereof, or a pharmaceutically acceptable salt thereof is administered to the subject twice daily at a dose of 30 mg to 75 mg each time.

11. The method of any one of claims 1 to 10, wherein the compound of formula (I), a stereoisomer thereof, or a pharmaceutically acceptable salt thereof is administered to the subject twice daily at a dose of 45 mg to 60 mg each time.

12. The method of any one of claims 1 to 11, wherein the compound of formula (I), a stereoisomer thereof, or a pharmaceutically acceptable salt thereof has a unit dosage of 2.5 mg to 150 mg.

13. The method of any one of claims 1 to 12, wherein the compound of formula (I), a stereoisomer thereof, or a pharmaceutically acceptable salt thereof has a unit dosage of 15 mg to 150 mg.

14. The method of any one of claims 1 to 13, wherein the compound of formula (I), a stereoisomer thereof, or a pharmaceutically acceptable salt thereof has a unit dosage of 15 mg, 30 mg, 45 mg, 60 mg, 75 mg, 90 mg, 120 mg or 150 mg.

15. The method of any one of claims 1 to 14, wherein the compound of formula (I), a stereoisomer thereof, or a pharmaceutically acceptable salt thereof is administered to the subject in a tablet or capsule form.

16. The method of any one of claims 1 to 15, wherein the compound of formula (I), a stereoisomer thereof, or a pharmaceutically acceptable salt thereof is orally administered to the subject.

17. The method of any one of claims 1 to 16, wherein the subject is a mammal, preferably a human.

18. The method of any one of claims 1 to 17, wherein the subject suffers from latent tuberculosis infection.

19. The method of any one of claims 1 to 18, wherein the subject has a medical history of pulmonary tuberculosis infection, tuberculosis, and/or tuberculous pleurisy.

20. The method of any one of claims 1 to 19, wherein the subject suffers from latent tuberculosis infection, and latent tuberculosis infection is not reactivated after treatment.

21. The method of any one of claims 1 to 19, wherein the subject has a medical history of pulmonary tuberculosis infection, tuberculosis, and/or tuberculous pleurisy, and pulmonary tuberculosis infection, tuberculosis, and/or tuberculous pleurisy is/are not reactivated after treatment.

22. The method of any one of claims 1 to 19, wherein mucocutaneous manifestations of oral ulcers in the subject are improved.

23. The method of any one of claims 1 to 19, wherein the area under the curve of oral ulcers in the subject is reduced.

24. The method of any one of claims 1 to 19, wherein the number of oral ulcers in the subject is reduced.

25. The method of any one of claims 1 to 19, wherein a time required for oral ulcer healing is shortened, and/or recurrence of oral ulcers is reduced, and/or duration of complete remission of oral ulcers is prolonged.

26. The method of any one of claims 1 to 19, wherein oral ulcer pain in the subject is alleviated.

27. The method of any one of claims 1 to 19, wherein genital ulcers in the subject are improved, and/or genital ulcer pain in the subject is alleviated.

28. The method of any one of claims 1 to 19, wherein mucocutaneous involvement in the subject is improved.

29. The method of any one of claims 1 to 19, wherein joint involvement in the subject is improved.

30. The method of any one of claims 1 to 19, wherein the treatment of the subject does not require titrated dosing.

31. The method of any one of claims 1 to 19, wherein anxiety or depression in the subject is not induced or exacerbated.

32. The method of any one of claims 1 to 19, wherein immunity in the subject is not diminished.

33. A compound of formula (I), a stereoisomer thereof, or a pharmaceutically acceptable salt thereof for use in the treatment of Behçet's syndrome in a subject,

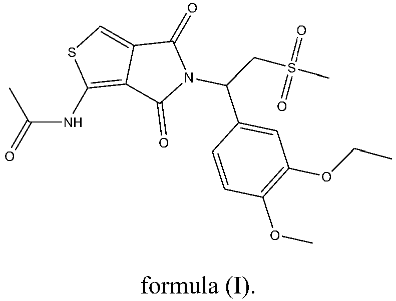

34. A compound of formula (I), a stereoisomer thereof, or a pharmaceutically acceptable salt thereof for use in the treatment of oral ulcers associated with Behçet's syndrome in a subject,

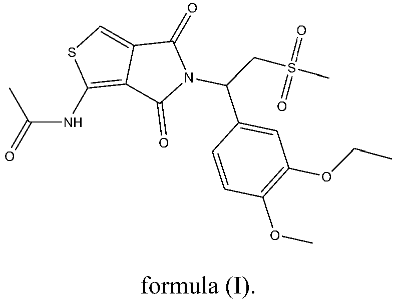

35. A compound of formula (I), a stereoisomer thereof, or a pharmaceutically acceptable salt thereof for use in the treatment of genital ulcers associated with Behçet's syndrome in a subject,

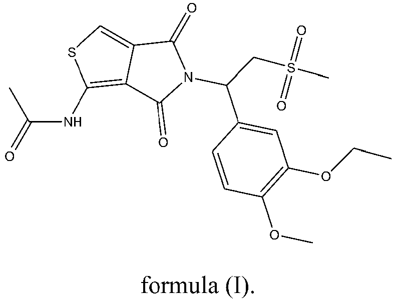

36. The compound of formula (I), a stereoisomer thereof, or a pharmaceutically acceptable salt thereof for use of any one of claims 33 to 35, wherein the compound of formula (I), a stereoisomer thereof, or a pharmaceutically acceptable salt thereof is administered to the subject daily at a dose of 30 mg to 180 mg.

37. The compound of formula (I), a stereoisomer thereof, or a pharmaceutically acceptable salt thereof for use of any one of claims 33 to 36, wherein the compound of formula (I), a stereoisomer thereof, or a pharmaceutically acceptable salt thereof is administered to the subject daily at a dose of 60 mg to 150 mg.

38. The compound of formula (I), a stereoisomer thereof, or a pharmaceutically acceptable salt thereof for use of any one of claims 33 to 37, wherein the compound of formula (I), a stereoisomer thereof, or a pharmaceutically acceptable salt thereof is administered to the subject daily at a dose of 150 mg.

39. The compound of formula (I), a stereoisomer thereof, or a pharmaceutically acceptable salt thereof for use of any one of claims 33 to 38, wherein the compound of formula (I), a stereoisomer thereof, or a pharmaceutically acceptable salt thereof is administered to the subject daily at a dose of 120 mg.

40. The compound of formula (I), a stereoisomer thereof, or a pharmaceutically acceptable salt thereof for use of any one of claims 33 to 39, wherein the compound of formula (I), a stereoisomer thereof, or a pharmaceutically acceptable salt thereof is administered to the subject at least once daily.

41. The compound of formula (I), a stereoisomer thereof, or a pharmaceutically acceptable salt thereof for use of any one of claims 33 to 40, wherein the compound of formula (I), a stereoisomer thereof, or a pharmaceutically acceptable salt thereof is administered to the subject at least twice daily.

42. The compound of formula (I), a stereoisomer thereof, or a pharmaceutically acceptable salt thereof for use of any one of claims 33 to 41, wherein the compound of formula (I), a stereoisomer thereof, or a pharmaceutically acceptable salt thereof is administered to the subject twice daily at a dose of 30 mg to 75 mg each time.

43. The compound of formula (I), a stereoisomer thereof, or a pharmaceutically acceptable salt thereof for use of any one of claims 33 to 42, wherein the compound of formula (I), a stereoisomer thereof, or a pharmaceutically acceptable salt thereof is administered to the subject twice daily at a dose of 45 mg to 60 mg each time.

44. The compound of formula (I), a stereoisomer thereof, or a pharmaceutically acceptable salt thereof for use of any one of claims 33 to 43, wherein the compound of formula (I), a stereoisomer thereof, or a pharmaceutically acceptable salt thereof has a unit dosage of 2.5 mg to 150 mg.

45. The compound of formula (I), a stereoisomer thereof, or a pharmaceutically acceptable salt thereof for use of any one of claims 33 to 44, wherein the compound of formula (I), a stereoisomer thereof, or a pharmaceutically acceptable salt thereof has a unit dosage of 15 mg to 150 mg.

46. The compound of formula (I), a stereoisomer thereof, or a pharmaceutically acceptable salt thereof for use of any one of claims 33 to 45, wherein the compound of formula (I), a stereoisomer thereof, or a pharmaceutically acceptable salt thereof has a unit dosage of 15 mg, 30 mg, 45 mg, 60 mg, 75 mg, 90 mg, 120 mg or 150 mg.

47. The compound of formula (I), a stereoisomer thereof, or a pharmaceutically acceptable salt thereof for use of any one of claims 33 to 46, wherein the compound of formula (I), a stereoisomer thereof, or a pharmaceutically acceptable salt thereof is administered to the subject in a tablet or capsule form.

48. The compound of formula (I), a stereoisomer thereof, or a pharmaceutically acceptable salt thereof for use of any one of claims 33 to 47, wherein the compound of formula (I), a stereoisomer thereof, or a pharmaceutically acceptable salt thereof is orally administered to the subject.

49. The compound of formula (I), a stereoisomer thereof, or a pharmaceutically acceptable salt thereof for use of any one of claims 33 to 48, wherein the subject is a mammal, preferably a human.

50. The compound of formula (I), a stereoisomer thereof, or a pharmaceutically acceptable salt thereof for use of any one of claims 33 to 49, wherein the subject suffers from latent tuberculosis infection.

51. The compound of formula (I), a stereoisomer thereof, or a pharmaceutically acceptable salt thereof for use of any one of claims 33 to 50, wherein the subject has a medical history of pulmonary tuberculosis infection, tuberculosis, and/or tuberculous pleurisy.

52. The compound of formula (I), a stereoisomer thereof, or a pharmaceutically acceptable salt thereof for use of any one of claims 33 to 50, wherein the subject suffers from latent tuberculosis infection, and latent tuberculosis infection is not reactivated after treatment.

53. The compound of formula (I), a stereoisomer thereof, or a pharmaceutically acceptable salt thereof for use of any one of claims 33 to 50, wherein the subject has a medical history of pulmonary tuberculosis infection, tuberculosis, and/or tuberculous pleurisy, and pulmonary tuberculosis infection, tuberculosis, and/or tuberculous pleurisy is/are not reactivated after treatment.

54. The compound of formula (I), a stereoisomer thereof, or a pharmaceutically acceptable salt thereof for use of any one of claims 33 to 50, wherein mucocutaneous manifestations of oral ulcers in the subject are improved.

55. The compound of formula (I), a stereoisomer thereof, or a pharmaceutically acceptable salt thereof for use of any one of claims 33 to 50, wherein the area under the curve of oral ulcers in the subject is reduced.

56. The compound of formula (I), a stereoisomer thereof, or a pharmaceutically acceptable salt thereof for use of any one of claims 33 to 50, wherein the number of oral ulcers in the subject is reduced.

57. The compound of formula (I), a stereoisomer thereof, or a pharmaceutically acceptable salt thereof for use of any one of claims 33 to 50, wherein a time required for oral ulcer healing is shortened, and/or recurrence of oral ulcers is reduced, and/or duration of complete remission of oral ulcers is prolonged.

58. The compound of formula (I), a stereoisomer thereof, or a pharmaceutically acceptable salt thereof for use of any one of claims 33 to 50, wherein oral ulcer pain in the subject is alleviated.

59. The compound of formula (I), a stereoisomer thereof, or a pharmaceutically acceptable salt thereof for use of any one of claims 33 to 50, wherein genital ulcers in the subject are improved, and/or genital ulcer pain in the subject is alleviated.

60. The compound of formula (I), a stereoisomer thereof, or a pharmaceutically acceptable salt thereof for use of any one of claims 33 to 50, wherein mucocutaneous involvement in the subject is improved.

61. The compound of formula (I), a stereoisomer thereof, or a pharmaceutically acceptable salt thereof for use of any one of claims 33 to 50, wherein joint involvement in the subject is improved.

62. The compound of formula (I), a stereoisomer thereof, or a pharmaceutically acceptable salt thereof for use of any one of claims 33 to 50, wherein the treatment of the subject does not require titrated dosing.

63. The compound of formula (I), a stereoisomer thereof, or a pharmaceutically acceptable salt thereof for use of any one of claims 33 to 50, wherein anxiety or depression in the subject is not induced or exacerbated.

64. The compound of formula (I), a stereoisomer thereof, or a pharmaceutically acceptable salt thereof for use of any one of claims 33 to 50, wherein immunity in the subject is not diminished.

65. Use of a compound of formula (I), a stereoisomer thereof, or a pharmaceutically acceptable salt thereof in the preparation of a medicament for the treatment of Behçet's syndrome in a subject,

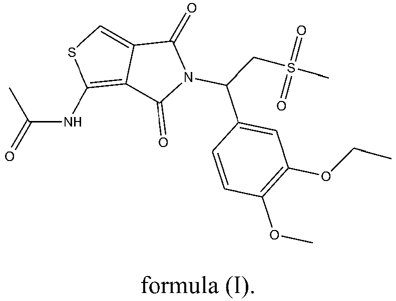

66. Use of a compound of formula (I), a stereoisomer thereof, or a pharmaceutically acceptable salt thereof in the preparation of a medicament for the treatment of oral ulcers associated with Behçet's syndrome in a subject,

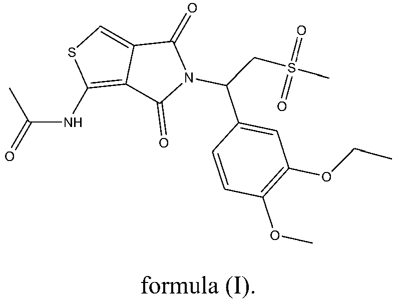

67. Use of a compound of formula (I), a stereoisomer thereof, or a pharmaceutically acceptable salt thereof in the preparation of a medicament for the treatment of genital ulcers associated with Behçet's syndrome in a subject,

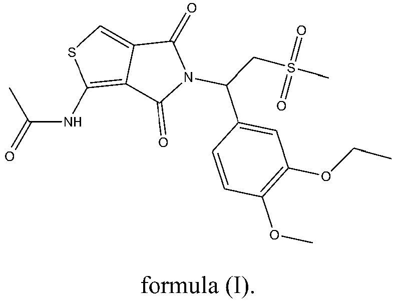

68. The use of any one of claims 65 to 67, wherein the compound of formula (I), a stereoisomer thereof, or a pharmaceutically acceptable salt thereof is administered to the subject daily at a dose of 30 mg to 180 mg.

69. The use of any one of claims 65 to 68, wherein the compound of formula (I), a stereoisomer thereof, or a pharmaceutically acceptable salt thereof is administered to the subject daily at a dose of 60 mg to 150 mg.

70. The use of any one of claims 65 to 69, wherein the compound of formula (I), a stereoisomer thereof, or a pharmaceutically acceptable salt thereof is administered to the subject daily at a dose of 150 mg.

71. The use of any one of claims 65 to 70, wherein the compound of formula (I), a stereoisomer thereof, or a pharmaceutically acceptable salt thereof is administered to the subject daily at a dose of 120 mg.

72. The use of any one of claims 65 to 71, wherein the compound of formula (I), a stereoisomer thereof, or a pharmaceutically acceptable salt thereof is administered to the subject at least once daily.

73. The use of any one of claims 65 to 72, wherein the compound of formula (I), a stereoisomer thereof, or a pharmaceutically acceptable salt thereof is administered to the subject at least twice daily.

74. The use of any one of claims 65 to 73, wherein the compound of formula (I), a stereoisomer thereof, or a pharmaceutically acceptable salt thereof is administered to the subject twice daily at a dose of 30 mg to 75 mg each time.

75. The use of any one of claims 65 to 74, wherein the compound of formula (I), a stereoisomer thereof, or a pharmaceutically acceptable salt thereof is administered to the subject twice daily at a dose of 45 mg to 60 mg each time.

76. The use of any one of claims 65 to 75, wherein the compound of formula (I), a stereoisomer thereof, or a pharmaceutically acceptable salt thereof has a unit dosage of 2.5 mg to 150 mg.

77. The use of any one of claims 65 to 76, wherein the compound of formula (I), a stereoisomer thereof, or a pharmaceutically acceptable salt thereof has a unit dosage of 15 mg to 150 mg.

78. The use of any one of claims 65 to 77, wherein the compound of formula (I), a stereoisomer thereof, or a pharmaceutically acceptable salt thereof has a unit dosage of 15 mg, 30 mg, 45 mg, 60 mg, 75 mg, 90 mg, 120 mg or 150 mg.

79. The use of any one of claims 65 to 78, wherein the compound of formula (I), a stereoisomer thereof, or a pharmaceutically acceptable salt thereof is administered to the subject in a tablet or capsule form.

80. The use of any one of claims 65 to 79, wherein the compound of formula (I), a stereoisomer thereof, or a pharmaceutically acceptable salt thereof is orally administered to the subject.

81. The use of any one of claims 65 to 80, wherein the subject is a mammal, preferably a human.

82. The use of any one of claims 65 to 81, wherein the subject suffers from latent tuberculosis infection.

83. The use of any one of claims 65 to 82, wherein the subject has a medical history of pulmonary tuberculosis infection, tuberculosis, and/or tuberculous pleurisy.

84. The use of any one of claims 65 to 82, wherein the subject suffers from latent tuberculosis infection, and latent tuberculosis infection is not reactivated after treatment.

85. The use of any one of claims 65 to 82, wherein the subject has a medical history of pulmonary tuberculosis infection, tuberculosis, and/or tuberculous pleurisy, and pulmonary tuberculosis infection, tuberculosis, and/or tuberculous pleurisy is/are not reactivated after treatment.

86. The use of any one of claims 65 to 82, wherein mucocutaneous manifestations of oral ulcers in the subject are improved.

87. The use of any one of claims 65 to 82, wherein the area under the curve of oral ulcers in the subject is reduced.

88. The use of any one of claims 65 to 82, wherein the number of oral ulcers in the subject is reduced.

89. The use of any one of claims 65 to 82, wherein a time required for oral ulcer healing is shortened, and/or recurrence of oral ulcers is reduced, and/or duration of complete remission of oral ulcers is prolonged.

90. The use of any one of claims 65 to 82, wherein oral ulcer pain in the subject is alleviated.

91. The use of any one of claims 65 to 82, wherein manifestations of genital ulcers in the subject are improved, and/or genital ulcer pain in the subject is alleviated.

92. The use of any one of claims 65 to 82, wherein mucocutaneous involvement in the subject is improved.

93. The use of any one of claims 65 to 82, wherein joint involvement in the subject is improved.

94. The use of any one of claims 65 to 82, wherein the treatment of the subject does not require titrated dosing.

95. The use of any one of claims 65 to 82, wherein anxiety or depression in the subject is not induced or exacerbated.

96. The use of any one of claims 65 to 82, wherein immunity in the subject is not diminished.
